(19)

**Europäisches Patentamt**
**European Patent Office**
**Office européen des brevets**

(11) **EP 4 353 833 B1**

(12) **EUROPEAN PATENT SPECIFICATION**

(45) Date of publication and mention
of the grant of the patent:
**08.10.2025 Bulletin 2025/41**

(21) Application number: **22200681.9**

(22) Date of filing: **10.10.2022**

(51) International Patent Classification (IPC):
***C12Q 1/6825*** (2018.01)     ***C12Q 1/6839*** (2018.01)

(52) Cooperative Patent Classification (CPC):
(C-Sets available)
**C12Q 1/6825; C12Q 1/6839**     (Cont.)

(54) **ORIGAMI-FINGERPRINTING BASED DIAGNOSTICS**

DIAGNOSE AUF ORIGAMI-FINGERABDRUCKBASIS

DIAGNOSTICS BASÉS SUR L'IMPRESSION AU DOIGT D'ORIGAMI

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**17.04.2024 Bulletin 2024/16**

(73) Proprietor: **University Of Fribourg
1700 Fribourg (CH)**

(72) Inventors:
• **Domljanovic, Ivana**
**3013 Bern (CH)**
• **Kocabey, Samet**
**1700 Fribourg (CH)**
• **Rüegg, Curzio**
**1630 Bulle (CH)**

(74) Representative: **Stolmár & Partner
Patentanwälte PartG mbB
Blumenstraße 17
80331 München (DE)**

(56) References cited:
**WO-A1-2022/072850     US-A1- 2016 082 122**

• LORETAN MORGANE ET AL: "DNA Origami as Emerging Technology for the Engineering of Fluorescent and Plasmonic-Based Biosensors", MATERIALS, vol. 13, no. 9, 9 May 2020 (2020-05-09), pages 2185, XP055871225, Retrieved from the Internet <URL:https://www.ncbi.nlm.nih.gov/pmc/articles/PMC7254321/pdf/materials-13-02185.pdf> DOI: 10.3390/ma13092185
• BELLASSAI NOEMI ET AL: "Novel nucleic acid origami structures and conventional molecular beacon-based platforms: a comparison in biosensing applications", ANALYTICAL AND BIOANALYTICAL CHEMISTRY, SPRINGER BERLIN HEIDELBERG, BERLIN/HEIDELBERG, vol. 413, no. 24, 6 April 2021 (2021-04-06), pages 6063 - 6077, XP037563258, ISSN: 1618-2642, [retrieved on 20210406], DOI: 10.1007/S00216-021-03309-4
• WANG SHUANG ET AL: "DNA Origami-Enabled Biosensors", SENSORS, vol. 20, no. 23, 3 December 2020 (2020-12-03), CH, pages 6899, XP093030240, ISSN: 1424-8220, DOI: 10.3390/s20236899

Remarks:
The complete document including Reference Table(s) and the Sequence Listing(s) can be downloaded from the EPO website

EP 4 353 833 B1

(52)  Cooperative Patent Classification (CPC): (Cont.)

C-Sets
**C12Q 1/6825, C12Q 2565/133, C12Q 2565/549;
C12Q 1/6839, C12Q 2565/133, C12Q 2565/549**

**Description**

**Field of the invention**

The present invention is directed to DNA origami-based biosensors capable of detecting target oligonucleotides.

**Background**

Cancer-associated nucleic acids, including circulating cell-free, tumour-derived DNA fragments (ctDNAs) carrying tumour-specific sequence alterations or circulating microRNAs (miRNAs), are increasingly considered as potential cancer biomarkers for use in non-invasive early cancer diagnostics and disease monitoring strategies. These nucleic acids are present in low abundance in the blood of cancer patients and their concentrations and profiles are significantly different in comparison to those of healthy individuals, making them ideal tumour biomarkers.

Currently, ctDNA and miRNAs are typically detected by quantitative real-time PCR (qRT-PCR), next-generation sequencing (NGS), or microarray hybridization techniques. Regardless of their enormous analytical progress, these methods have some limitations. Most typically, they are complex, involve multiple steps, demand expensive instrumentation, and are time-consuming. In addition, methods based on direct oligonucleotide hybridization may be hampered by the difficulty to discriminate ctDNA fragments differing only by single (point) mutations. This is also the case for highly homologous miRNAs that differ by only one single base which makes it equally difficult to discriminate between different family members.

Importantly, detection of multiple biological molecules in complex conditions is becoming increasingly frequent for early diagnosis and monitoring. Therefore, there is a growing and unfulfilled need for more efficient approaches and tools to detect cancer-associated nucleic acids with high sensitivity, sequence specificity, and high output for use in clinical and medical routines. These facts lead to a growing demand for point-of-care diagnostic nanoscale devices that are easy to use and cost-effective alternatives to expensive diagnostic devices, but also highly sensitive and specific.

DNA nanotechnology, especially DNA origami, enables the generation of nanoscale-sized structures with precisely defined geometries and functional modalities that can be engineered into highly specific, rapid, high throughput analytical devices with single-molecule sensitivity for biomedical applications. Importantly, DNA origami allows the engineering of "dynamic" structures, whose function depends on conformational changes induced by external stimuli such as DNA-based strand-displacement reactions. Conformational changes can thus generate a measurable signal that can be exploited to sense the external stimuli. The measurable signal can be detected by optical and electrochemical techniques. In case of optical detection, DNA origami structures can be functionalized with fluorophores.

One intrinsic feature of DNA self-assembly is the ability to generate precise and predetermined three-dimensional arrangement of fluorophore networks to produce strong optical signals. This precise arrangement of multiple fluorophores can be used to create Förster resonance energy transfer (FRET) and/or quenching networks. These FRET-based DNA photonic networks are especially relevant for multiplex detection applications. One of the first DNA origami structures created to detect small oligonucleotides (viral RNA or miRNA) was reported by Andersen et al. (E. S. Andersen, M. Dong, et al., Nature, 2009, 459, 73-76). With the advances in the field of nanotechnology, in particular, the development of the DNA origami method, more complex and advanced structures were generated that were able to detect low concentrations of nucleic acids. WO 2022/072850 A1 discloses a DNA origami biosensor, comprising a DNA origami hinge (which is a scaffold strand) and staple strands and overhang staple strands. It includes a zippered latch (lock) that consists of a latch strand and the staples, the latch strand has a toehold domain. When the target binds to the toehold domain and displaces the staple fastening strands from the rest of the latch strand, the hinge is in an open configuration. Each of the arms of the hinge can be a multilayered structure, and the staple strands can be bound to fluorescence reporters, which are activated depending on the binding of target and associated opening of the lock.

However, previously described structures are only able to detect one kind of nucleic acids. Given the multitude of nucleic acids that need to be detected, there is therefore a need for DNA origami structures capable of detecting the presence of different nucleic acids at the same time.

**Summary**

In one aspect, the invention is directed to a DNA origami biosensor capable of detecting at least two different target oligonucleotides at the same time, said biosensor comprising at least one DNA origami hinge and at least two detection sites, wherein each detection site comprises

- a signal emitting array,
- at least one lock comprising a first and a second fastening domain and a toehold domain attached to the first or second fastening domain, wherein the first and the second fastening domain are capable of forming a duplex with each other and wherein the first or second fastening domain is capable of hybridizing to a target oligonucleotide via the toehold domain, wherein the lock is capable of assuming an open or a locked configuration, wherein the lock assumes the open configuration upon hybridization of the target oligonucleotide and the locked configuration in the absence of the target oligonucleotide,

- at least one movable section, wherein the movable section is connected to the at least one hinge and is movable via said hinge and wherein the position of the movable section depends on the configuration of the lock,

wherein the movable sections of the detection sites are connected to each other or to a common DNA origami structure via the at least one hinge.

In another aspect, the invention is directed to a method for detecting at least two target oligonucleotides in a sample, the method comprising

a) bringing a biosensor according to the invention into contact with the sample.

In a third aspect, the invention is directed to a biosensor for use in diagnosing a disease, in particular a disease associated with altered miRNA expression, as well as methods of diagnosing such diseases.

**Brief description of the figures**

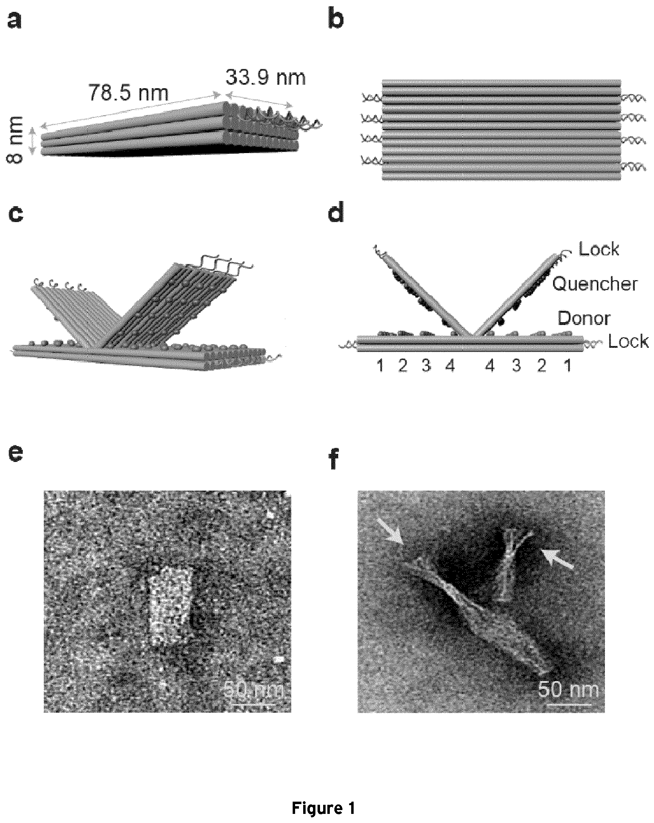

Fig. 1 shows the characterization of the DNA origami book biosensor according to one embodiment of the present invention. (a) Representation of the dimensions of the book biosensor in its closed state. (b) Top view of the book biosensor with 4 locks on each side. (c) Side view of the FRET biosensor in the open state. Spheres represent the donor and acceptor fluorophore arrays. In total, 20 FRET pairs were positioned on each side. (d) Side view of the quencher biosensor in the open state. Spheres represent the arrays of donor and quencher groups. In total, 20 fluorophore/quencher pairs were positioned on each side. Numbers (1-4) represent the positions of columns within the DNA origami structure. Electron micrographs of DNA origami structures assembled in the (e) closed conformation and (f) open conformation at 12 mM $Mg^{2+}$ (scale bars 50 nm). Arrows show the opening of the upper layer at the sides of the structures.

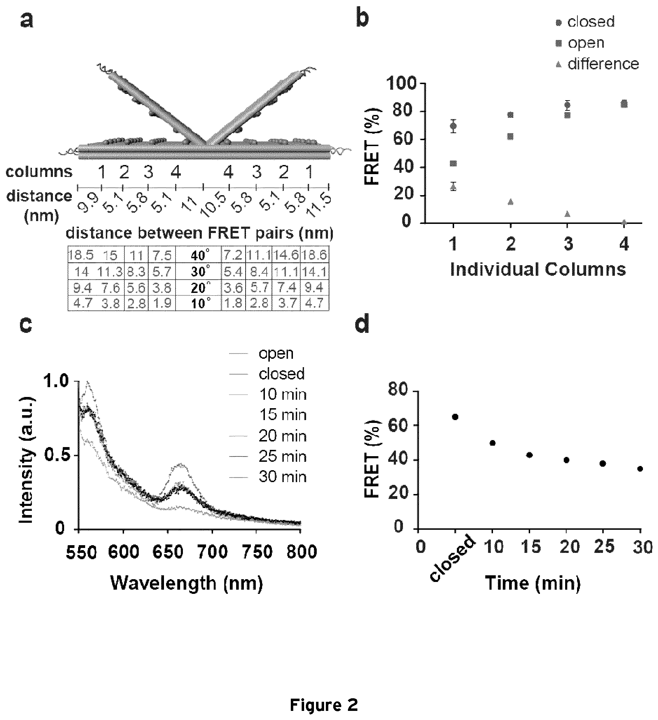

Fig. 2 shows the determination of FRET efficiency of the DNA origami book biosensor using fluorescence spectroscopy. (a) Calculations of the relative distance between FRET pairs at a theoretical angle of opening of the structure. (b) Fluorescence spectroscopy characterization of FRET performance of individual columns within the structure. (c) Spectroscopic characterization of the opening of DNA origami book biosensors with 5 FRET pairs. (d) Donor excited fluorescence spectra of acceptors at closed and open states of DNA origami book biosensors with 5 FRET pairs.

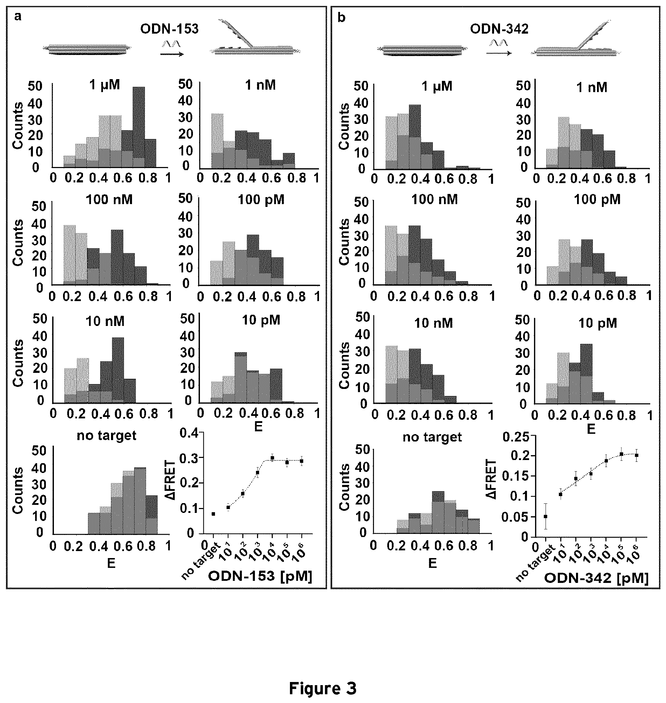

Fig. 3 shows the detection limit of the book biosensor with 3 columns (1-3) on each side at a single DNA origami level. (a) Opening at the left side of the DNA origami book upon addition of ODN-153. (b) Opening at the right side of the DNA origami book upon addition of ODN-342. The dark grey and light grey histograms represent FRET efficiency distribution before (0 min) and after (6 min) the addition of target and non-target (control) ODNs at different concentrations.

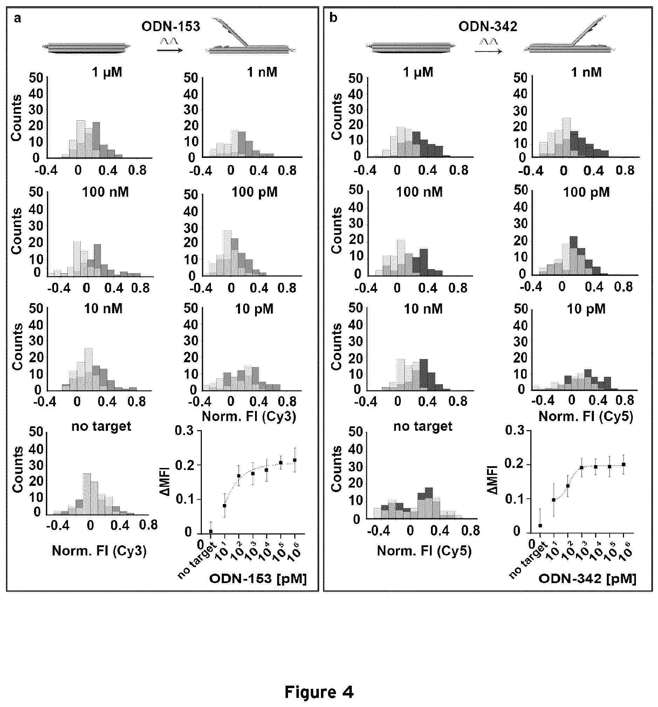

Fig. 4 shows the detection limit of the book biosensor with 2 columns (1-2) of dye-quencher pairs on each side at a single DNA origami device level. (a) Opening at the left side of the DNA origami book upon addition of ODN-153. (b) Opening at the right side of the DNA origami book upon addition of ODN-342. The dark grey histograms represent fluorescence intensity increase after the addition of the target of interest due to the opening of the structure. A light grey histogram represents fluorescence intensity in the closed state.

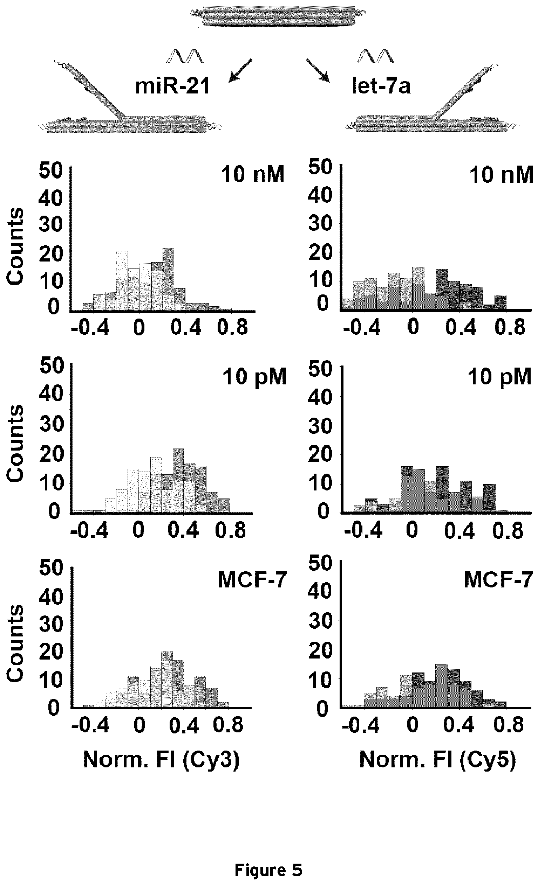

Fig. 5 shows the simultaneous detection of miR-21 and let-7a using the book biosensor with 2 columns (1-2) of dye-quencher pairs on each side. Left: Detection of miR-21 at 10 nM and 10 pM concentrations. Right: Detection of let-7a at 10 nM and 10 pM concentrations. Top and middle: Detection of synthetic miR-21 and let-7a. Bottom: Detection of miR-21 and let-7a from MCF-7 cell extract. The dark grey histograms represent fluorescence intensity increase after the addition of the target of interest due to the opening of the structure. A light grey histogram represents fluorescence intensity in the closed state.

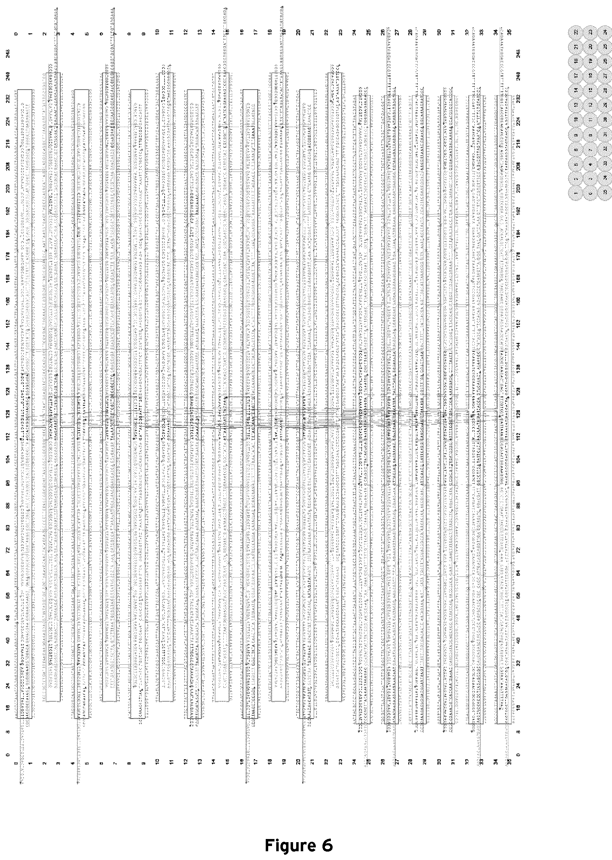

Fig. 6 shows the scaffold routing and staple design in a two-dimensional representation. Graphics and sequences were generated using caDNAno software package.

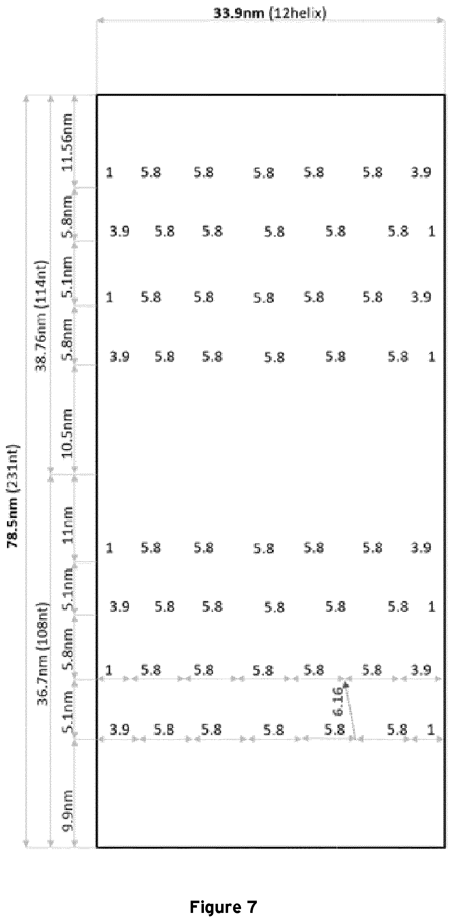

Fig. 7 shows the map of a DNA origami book biosensor showing the distance between the positioned fluorophores or quenchers.

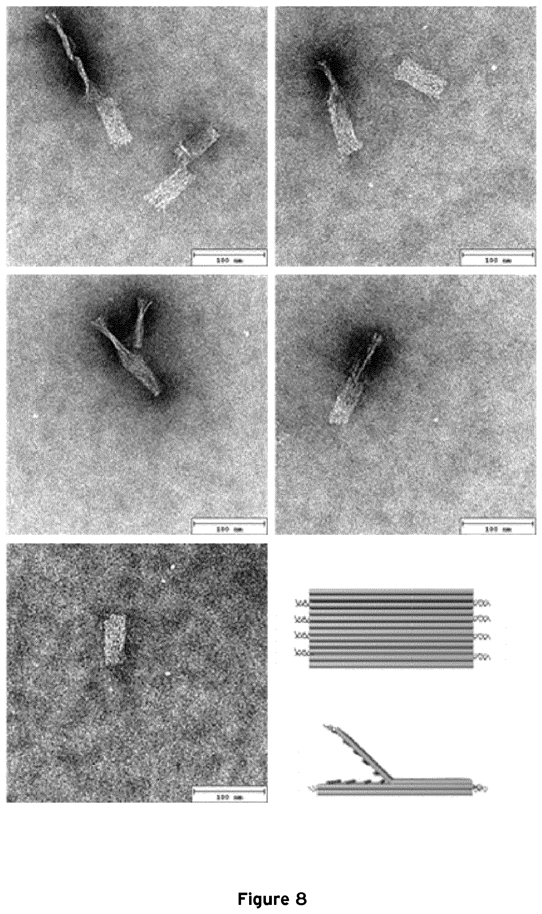

Fig. 8 shows TEM images of DNA origami book biosensors. Structures were self-assembled at 12 mM $Mg^{2+}$ and purified at 8 mM $Mg^{2+}$ and visualized using transmission electron microscopy (TEM) by depositing them on the carbon-coated EM grids (scale bars: 100 nm).

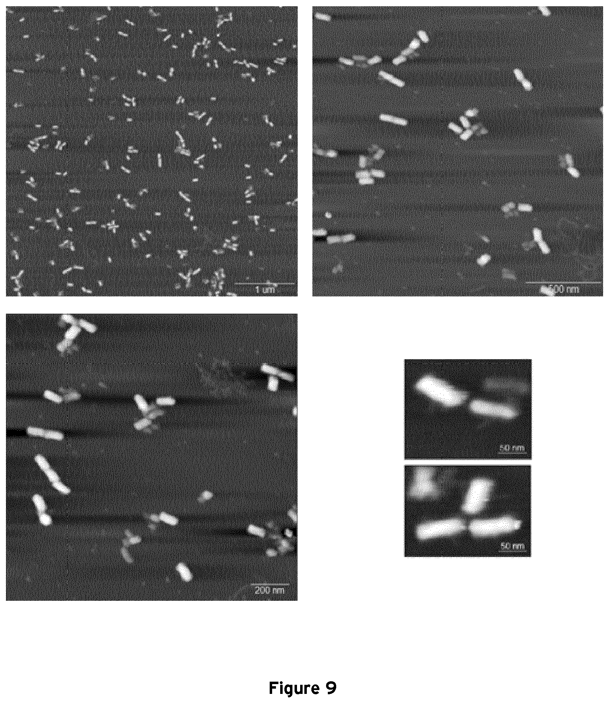

Fig. 9 shows DNA origami book biosensor visualization using atomic force microscopy (AFM). Structures were assembled with 12 mM $Mg^{2+}$ in the closed conformation and absorbed on the mica surface. AFM measurements were performed in tapping mode with AFM NT-MDT (NTEGRA II).

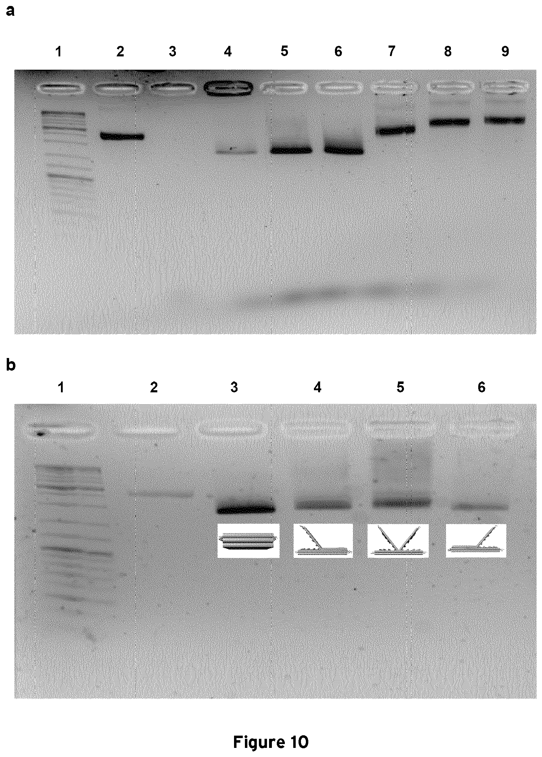

Fig. 10 shows an agarose gel analysis of DNA origami book biosensors. The presence of $Mg^{2+}$ is needed to shield the negatively charged DNA phosphate backbone, for the formation of DNA double helices during the self-assembly process of DNA origami structure. To determine the optimal $Mg^{2+}$ concentration for the self-assembly of the book biosensor, the structures were analysed by agarose gel electrophoresis. Assembled structures were run on 1.5% agarose gel at 70 V together with a 1 kb ladder (1), single-stranded p8064 scaffold strand (2) and staple mix (3). Gel electrophoresis analysis of the self-assembled structures with different $Mg^{2+}$ concentrations (4: 16 mM $Mg^{2+}$, 5: 12 mM $Mg^{2+}$, 6: 8 mM $Mg^{2+}$, 7: 4 mM $Mg^{2+}$, 8: 2 mM $Mg^{2+}$, 9: 1 mM $Mg^{2+}$). The optimal $Mg^{2+}$ concentration for forming the structure without incorporated fluorophore was greater than 8 mM $Mg^{2+}$ (Figure 10a). Figure 10b demonstrates the gel electrophoresis analysis of the self-assembled structures in the closed state (without fluorophores) at 12 mM $Mg^{2+}$ and purified at 8 mM $Mg^{2+}$ without (3) or with the addition of target oligonucleotides (4: ODN-153, 5: ODN-153+ODN-342 and 6: ODN-342). The gel image shows the differences in the mobility of the structures between open or closed states.

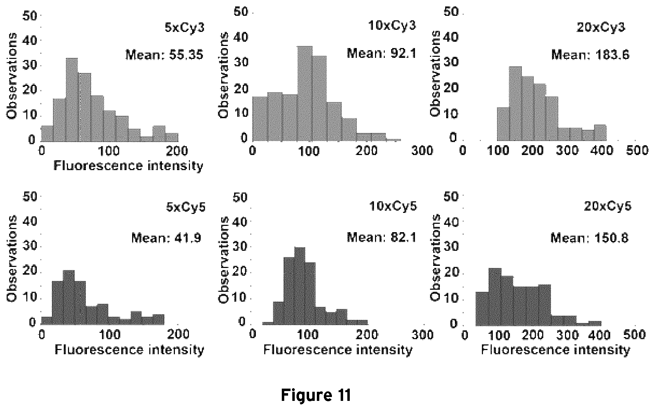

Fig. 11 shows a single-structure study of the incorporation of the fluorophores within DNA origami book biosensor using wide-field fluorescence microscopy. In order to test the incorporation of the dyes and measure the fluorescence signal generated by DNA origami book structures, the structures were functionalized with biotin to allow their immobilization on coverslips covered by biotinylated bovine serum albumin (BSA) and neutravidin. To determine if the incorporation of labelled oligonucleotides within the structure is efficient, we first analysed the DNA origami book structure with 1 column (5 Cy3 or Cy5), 2 columns (10 Cy3 or Cy5) and 4 columns (20 Cy3 or Cy5). Structures were imaged and the mean intensity of each structure was compared. The increase in the number of fluorophores incorporated within the structure resulted in a linear increase of the mean fluorescence intensity in the absence of self-quenching.

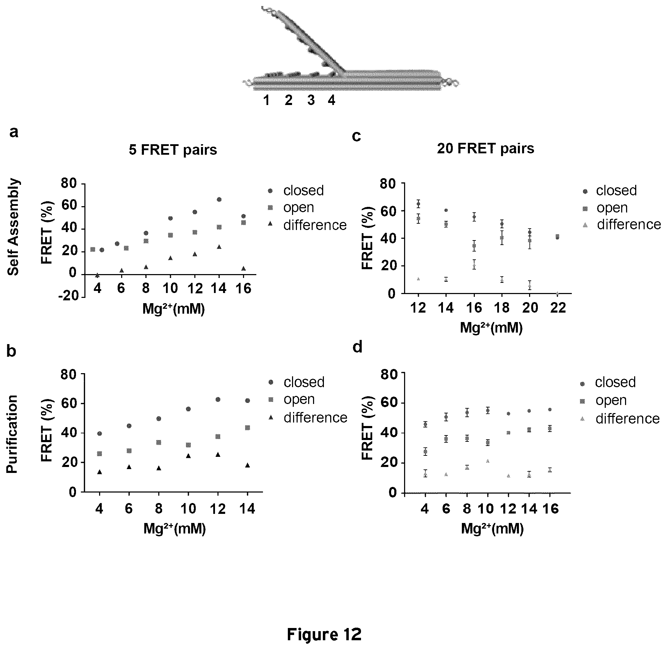

Fig. 12 shows the fluorescence spectroscopy analysis of FRET efficiency. To determine the optimal $Mg^{2+}$ concentration during the self-assembly and purification steps that give the maximum signal difference between open and closed states, we applied the FRET-based detection mechanism by labeling oligonucleotides with Cy3 and Cy5 fluorophores using terminal transferase (TdT) enzyme and incorporated them into DNA origami structure. Ensemble FRET analysis of the DNA origami book biosensors using fluorescence spectroscopy was performed. FRET efficiency of open and closed self-assembled structures with 5 FRET pairs at different $Mg^{2+}$ concentrations (4-16 mM $Mg^{2+}$) is shown in Figure 12a. Results indicated the optimal signal and the maximum difference between open and closed states (24%) observed when the structure is folded in the presence of 14 mM $Mg^{2+}$. After defining the optimal salt concentration during self-assembly that gives the maximum signal difference, the optimal $Mg^{2+}$ concentration for purification was determined. All structures were assembled at 14 mM $Mg^{2+}$ in the open and closed states and purified in the presence of $Mg^{2+}$ in the range of 4 mM to 14 mM (Figure 12b). The optimal purification concentration was defined as 10 mM, as it gave the maximum FRET difference between the open and closed states of the device (25%). Higher concentrations of salt are used during self-assembly to decrease repulsion between DNA helices facilitating keeping the DNA origami sensor in the closed state. On the other hand, the lower salt concentration used in the purification step increases the repulsion between DNA helices, which allows the opening of the structure upon ligand binding. We also tested if a higher number of fluorophores incorporated within the structure requires more $Mg^{2+}$. DNA origami structure with 4 columns (20 fluorophores) was assembled in open and closed states in the range of 12 to 22 mM $Mg^{2+}$ and the optimal signal was found at 16-18 mM $Mg^{2+}$ (Figure 12c), while the purification step was optimal in the range of 10-12 mM $Mg^{2+}$ (Figure 12d).

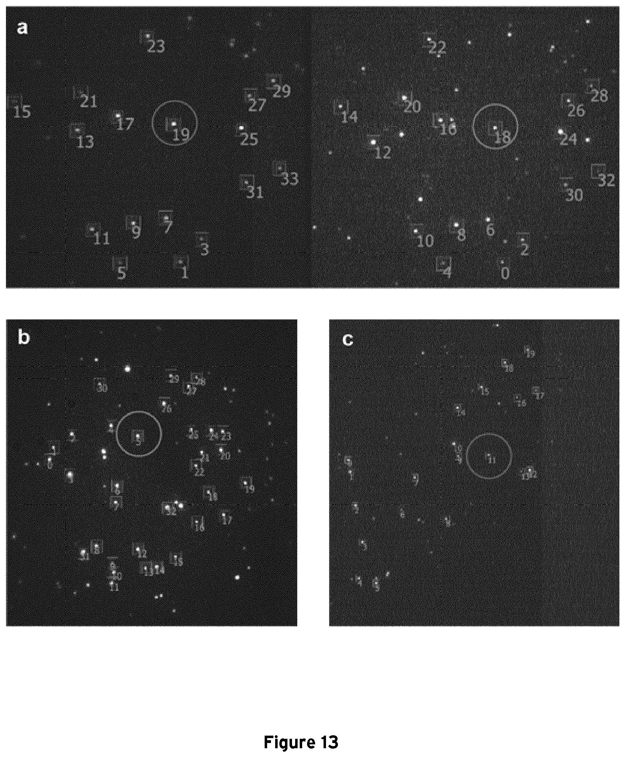

Fig. 13 shows a single-structure analysis of the DNA origami book biosensor. Fluorescence images were recorded 6 min after the addition of the target. (a) A representative image of the FRET-based detection method. The image shows the emission profiles of single DNA origami book sensors after donor excitation at 532 nm; the light grey circle and dark grey circle represent Cy3 and Cy5 emissions for the same DNA origami book biosensor, respectively. (b) A representative image of the quenching-based detection method (Cy3+bh2). It shows the emission after excitation at 532 nm; the light grey circle represents a single DNA origami structure. (c) A representative image of the quenching-based detection method (Cy5+bbq650). It shows the emission after excitation at 640 nm; the dark grey circle represents a single DNA origami structure.

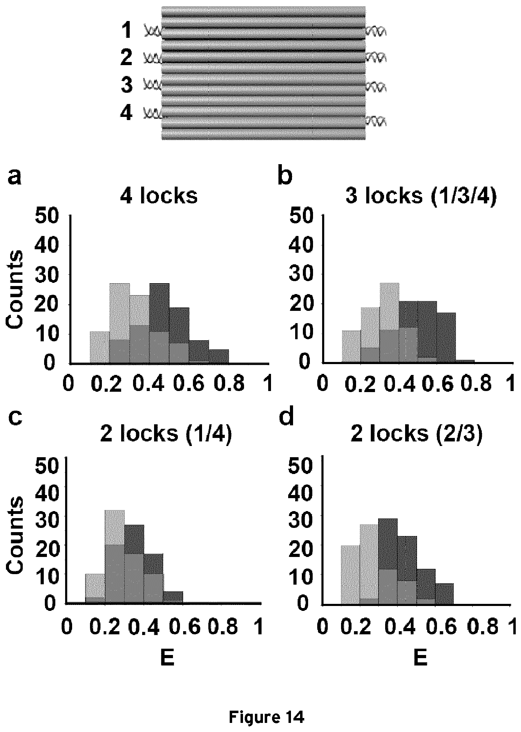

Fig. 14 shows the effect of amount and position of the locks within DNA origami book biosensor on FRET efficiency using wide-field fluorescence microscopy. Schematic representation of the positioning of locks within DNA origami book biosensor. Four different structures with 15 FRET pairs were assembled with different combinations of locks: (a) combination of all four locks, (b) with 3 locks (1, 3 and 4), (c) 2 locks at the edges (1 and 4) and (d) 2 locks in the middle (2 and 3). Detection of 100 pM of target DNA, ODN-153 was performed using these structures. Results were plotted into histograms where dark grey and light grey represent FRET efficiency distribution before (0 min) and after (6 min) the addition of target DNA.

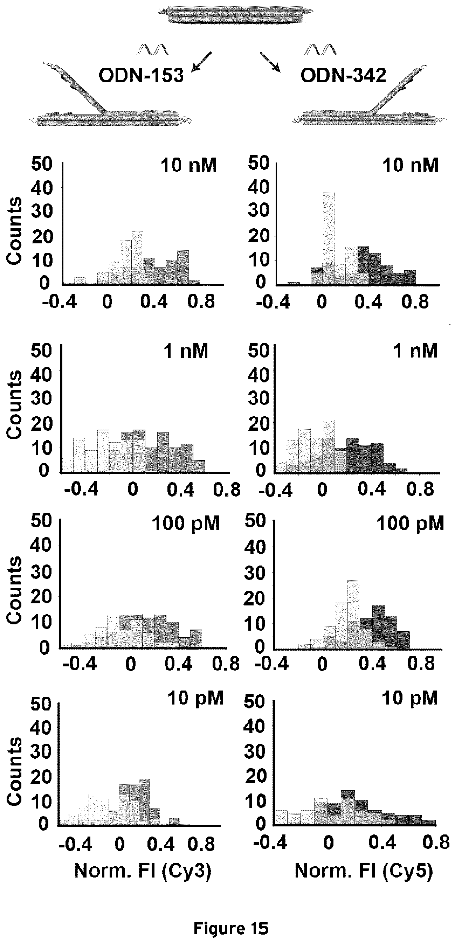

Fig. 15 shows a single-structure study of the limit of detection for multisensing of DNA targets. The detection limit of the book biosensor with 10 quenching pairs on both sides and with the addition of 2 DNA targets at the same time. Left: Opening at the left side of DNA origami book upon addition of ODN-153. Right: Opening at the right side of DNA origami book upon addition of ODN-342. The dark grey histograms represent fluorescence intensity increase after the addition of the target of interest due to the opening of the structure. A light grey histogram represents fluorescence intensity in the closed state.

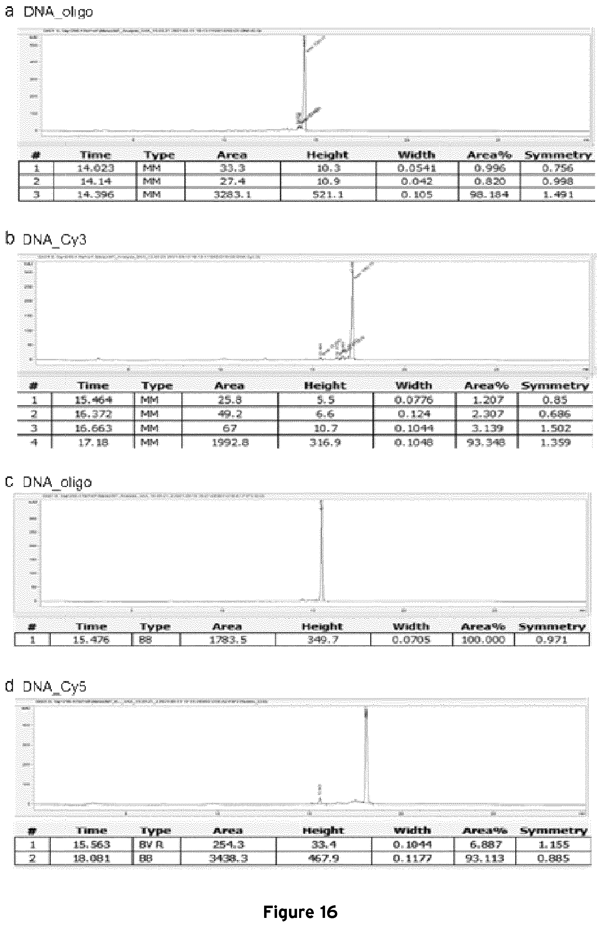

Fig. 16 shows terminal transferase (TdT) labeling of oligonucleotides with Cy3 and Cy5. Five oligonucleotides from each column were labelled with either Cy3 or Cy5-conjugated ddUTP using TdT enzyme. The efficiency of labeling was analysed using HPLC. Results showed that labeling of oligonucleotides with Cy3 or Cy5 was efficient with an approximate yield of 93%.

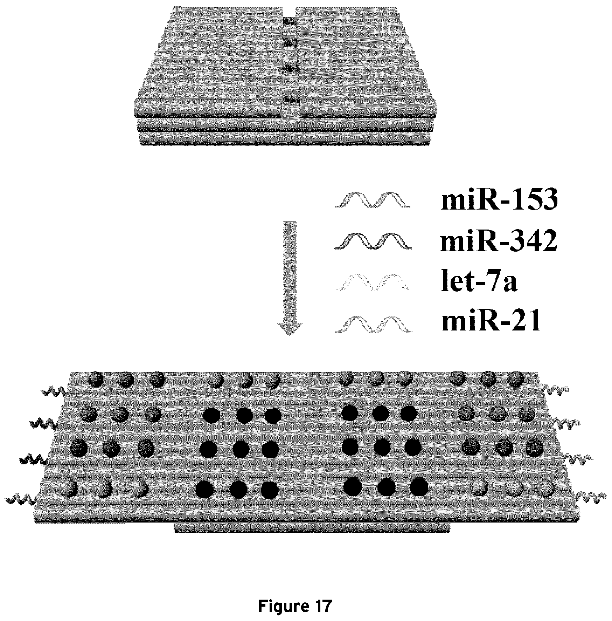

Fig. 17 shows the bridge structure. It consists of three layers where upper layer is connected to the middle layer with hinges on both ends to allow opening of the upper layer from the centre. This results in greater flexibility and ability to separate the helices as fingers to be able to detect four different biomarkers.

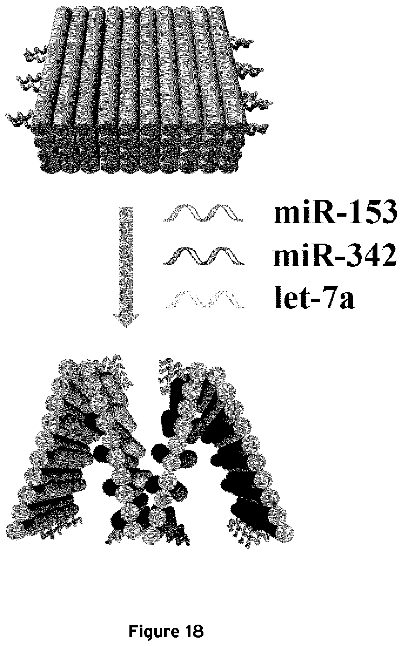

Fig. 18 shows the M structure. It consists of one layer of DNA helices that looks like a folded sheet of paper. For all three designs, hybridization-based locks on both sides constitutively keep all the layers in a closed state. The presence of target miRNA promotes the opening of the layers through displacement hybridization resulting in change of FRET signal of the fluorophore or increase fluorescence signal by releasing of the quenching.

Detailed description

In one embodiment, the invention is directed to a DNA origami biosensor capable of detecting at least two different target oligonucleotides at the same time, said biosensor comprising at least one DNA origami hinge and at least two detection sites, wherein each detection site comprises

- a signal emitting array,

- at least one lock comprising a first and a second fastening domain and a toehold domain attached to the first or second fastening domain, wherein the first and the second fastening domain are capable of forming a duplex with each other and wherein the first or second fastening domain is capable of hybridizing to a target oligonucleotide via the toehold domain, wherein the lock is capable of assuming an open or a locked configuration, wherein the lock assumes the open configuration upon hybridization of the target oligonucleotide and the locked configuration in the absence of the target oligonucleotide,

- at least one movable section, wherein the movable section is connected to the at least one hinge and is movable via said hinge and wherein the position of the movable section depends on the configuration of the lock,

wherein the movable sections of the detection sites are connected to each other or to a common DNA origami structure via the at least one hinge.

As used herein, the term "biosensor" refers an analytical tool consisting of biological components such as nucleic acids or proteins that is used to detect the presence of a target, generating a signal upon detection of the target.

According to the invention, the biosensor is capable of detecting the presence of target oligonucleotides, i.e. freely circulating oligonucleotides that are present in a sample.

DNA origami structures use DNA as a building material to form geometrical shapes on a nanoscale. DNA origami uses numerous short single-stranded nucleic acids termed "staple strands" which hybridize to portions of one or more long, single-stranded polynucleotides called "scaffold strand" to form particular pre-designed shapes (Rothemund, P. Folding DNA to create nanoscale shapes and patterns. Nature 440, 297-302 (2006). Douglas SM, Dietz H, Liedl T, Högberg B, Graf F, Shih WM. Self-assembly of DNA into nanoscale three-dimensional shapes. Nature 459 (7245), 414-418 (2009). Doerr, A. DNA origami in 3D. Nat Methods 8, 454 (2011). Han D, Pal S, Nangreave J, Deng Z, Liu Y, Yan H. DNA Origami with Complex Curvatures in Three-Dimensional Space. Science 332 (6027), 342-346 (2011)). The staple strands may contain additional nucleotides at either 5' or 3' end called overhangs that can be functionalized, for example biotinylated, modified with chemical moieties or designed to hybridize with target oligonucleotides.

The biosensor according to the invention comprises at least one DNA origami hinge. A hinge is herein defined as a structure that allows sections connected thereto to change their position, i.e. to move from one position to another. In some embodiments, the biosensor according to the invention comprises exactly one hinge. In some embodiments, the biosensor comprises exactly 2, 3, 4 or 5 hinges.

The biosensor according to the invention further comprises at least two detection sites. A detection site is herein defined as a site allowing the detection of a target oligonucleotide. A detection site is able to detect the presence of the target oligonucleotide via binding thereof to one of the locks of the detection sites, which results in a change in the position of the movable section and the emission of a signal from the signal emitting array.

A biosensor capable of detecting two different target oligonucleotides comprises two detection sites each capable of detecting a different target oligonucleotide, i.e. for each target oligonucleotide to be detected, the biosensor comprises a different detection site.

Each detection site comprises a signal emitting array. The signal emitted by the signal emitting arrays is a fluorescence signal. According to some embodiment, the fluorescence signal may be based on FRET (Förster resonance energy transfer) or quenching. A signal emitting array comprises at least one row of donor fluorophores opposing at least one row of acceptor fluorophores or at least one row of fluorophores opposing at least one row of quenchers. Preferably, a row of fluorophores or quenchers comprises 2, 3, 4, 5, 6, 7, 8, 9, 10, 15 or 20 fluorophores or quenchers. In a preferred embodiment, a row of fluorophores or quenchers comprises 5 fluorophores or quenchers. In some embodiments, a signal emitting array may comprise 2, 4, 6, 8 or 10 opposing rows of fluorophores or quenchers. In a preferred embodiment, the signal emitting arrays comprise 8 rows of fluorophores or quenchers opposing each other, i.e., four rows opposing four rows. In the context of the signal emitting array, the terms "row" and "column" are used interchangeably. The more fluorophores a signal emitting array comprises, the stronger the fluorescence signal is, thus facilitating the signal read-out.

The signal emitting arrays are constructed by labelling individual staple strands with fluorophores or quenchers. Examples of fluorophores and quenchers include Black Hole Quencher (BHQ), Black Hole Quencher-2 (bh2), BHQ3, FAM, AlexaFluor 488, AlexaFluor 555, AlexaFluor 647, Cy3, Cy5, quantum dots in the equivalent fluorophore wavelengths, Iowa Black RQ, Iowa Black FQ, Black-Berry Quencher 650 (bbq650), ATTO 488, ATTO 550, and 647N. The fluorophores and the quenchers used herein can be any fluorescent dye known in the art. In a preferred embodiment, Cy3 and Cy5 are used as donors and acceptor fluorophores, respectively. In another preferred example, Cy3 is used as fluorophore and bh2 or bb650q are used as quenchers.

Each detection area further comprises at least one lock comprising a first and a second fastening domain and a toehold domain attached to the first fastening domain, wherein the first and the second fastening domain are capable of forming a duplex with each other and wherein the first fastening domain is capable of hybridizing to a target oligonucleotide via the toehold domain, wherein the lock is capable of assuming an open or a locked configuration, wherein a lock assumes the open configuration upon hybridization of the target oligonucleotide and the locked configuration in the absence of the target oligonucleotide.

The first and the second fastening domain are overhangs of staple strands that are accessible for binding of target oligonucleotides. The first and second fastening domain of each lock are capable of hybridizing with each other in the absence of the target oligonucleotide and forming a duplex. That is, in the absence of its respective target oligonucleotide, the first and the second fastening domain of the lock may hybridize, so that the lock assumes a locked configuration.

If the target oligonucleotide is present, it may hybridize to the toehold domain and further to the first fastening domain, thereby replacing the second fastening domain from the duplex. The lock will then assume an open configuration.

Preferably, each detection area comprises several locks that are capable of hybridizing to the same target nucleotide. This enhances sensitivity and specificity of the biosensor.

Each detection site further comprises at least one movable section, wherein the movable section is connected to the at least one hinge and is movable via said hinge and wherein the position of the movable section depends on the configuration of the lock, i.e., when the lock assumes the open position, the movable section moves from a first position to a second position and when the lock assumes the closed position, the movable section moves back to the first position. Herein, the term "end of the movable section" refers to the end of the movable section distal to the hinge.

In a preferred embodiment, the first fastening domain of the lock is attached to the movable section of said detection area and the second fastening domain is attached to the common DNA structure or to the movable section of another detection area. Since the fastening domains of one lock are attached to different parts of the biosensor, the opening of the lock allows a change in the position of the movable section.

Each movable section comprises at least one row of fluorophores or quenchers of the signal emitting array belonging to the respective detection site. The opposing row or rows of fluorophores or quenchers of the signal emitting array may be located on the common DNA origami structure, e.g., on one of the rigid layers, or on the movable section of a different detection site. When the movable section moves from a first to a second position, the distance between the opposing rows of fluorophores or quenchers changes and thus a fluorescence signal is generated, increased or abolished.

In one embodiment, the biosensor comprises at least one rigid layer which serves as the common DNA origami structure. In some embodiments, the structure comprises 2, 3 or 4 rigid layers. The layers are located above each other. In a preferred embodiment, the common structure of the biosensor comprises 2 rigid layers.

In a preferred embodiment, the biosensor comprises at least one rigid layer and one DNA origami hinge, said hinge being connected to the at least one rigid layer and located in the middle of at least one rigid layer, wherein said hinge is connected to all movable sections of the biosensor. Preferably, the biosensor comprises two or four detection sites and two or four movable sections. The detection sites may be on the same or on different sides of the rigid layer. Such a biosensor having two detection sites on the same side of the rigid layer has the structure of a book. In this embodiment, the first fastening domains are located on the end of each movable section and the second fastening domains are located on both ends of the rigid layer.

In another embodiment, the biosensor comprises at least one rigid layer and two DNA origami hinges connected to the at least one rigid layer, the hinges being located at opposing ends of the at least one rigid layer, wherein each hinge is connected to at least one movable section. Preferably, the biosensor comprises two or four detection sites and two or four movable sections. The detection sites may be on the same or on different sides of the rigid layer. Such a biosensor having two detection sites on the same side of the rigid layer has the structure of a bascule bridge. In this embodiment, the first fastening domains are located on the end of each movable section and the second fastening domains are located in the middle of the rigid layer.

In yet another embodiment, the biosensor comprises no rigid layers, but three DNA origami hinges and three detection areas, wherein each detection area comprises two movable sections and wherein each hinge is connected to at least two movable sections. Such a biosensor has the structure of the letter M. In this embodiment, the fastening domains are located on both ends of each movable section.

All elements of the biosensor are formed by at least one scaffold strand and at least one staple strand, i.e. the biosensor comprises at least one scaffold strand and at least one staple strand. In one embodiment, the scaffold strand comprises a polynucleotide sequence that is at least 80%, at least 90%, at least 95%, or at least 99% identical to SEQ ID NO: 1.

In one embodiment, the staple strands comprise a polynucleotide sequence that is at least 80%, at least 90%, at least 95%, or at least 99% identical to any of SEQ ID NO: 2 to 120. These staple strands are also called the core staple (strands). They shape the scaffold and form the structure. In one embodiment, the core staples unmodified.

In one embodiment, the staple strands comprise a polynucleotide sequence that is at least 80%, at least 90%, at least 95%, or at least 99% identical to any of SEQ ID NO: 121 to 137. These staple strands are also called the foot staple (strands). They are at the center of the biosensor, in particular of the common DNA origami structure, and connect movable sections to the rigid layer.

In one embodiment, the staple strands comprise a polynucleotide sequence that is at least 80%, at least 90%, at least 95%, or at least 99% identical to any of SEQ ID NO: 138 to 149. These staple strands are also called the hinge staple (strands) and form the hinge.

In one embodiment, the staple strands comprise a polynucleotide sequence that is at least 80%, at least 90%, at least 95%, or at least 99% identical to any of SEQ ID NO: 150 to 155. In one embodiment, these staple strands are modified with biotin, thus enabling immobilization of the biosensor on a glass surface.

In one embodiment, the staple strands comprise a polynucleotide sequence that is at least 80%, at least 90%, at least 95%, or at least 99% identical to any of SEQ ID NO: 156 to 183. These staple strands are also called the endcap staple (strands) and are located at the end of the common DNA origami structure. They are extended with CCC sequence to prevent the nonspecific binding of single DNA origami structures to each other.

In one embodiment, the staple strands comprise a polynucleotide sequence that is at least 80%, at least 90%, at least 95%, or at least 99% identical to any of SEQ ID NO: 184 to 263. These staple strands form the rows of the signal emitting arrays to which the fluorophores or quenchers are attached. In a preferred embodiment, these staple strands are used as shown in the table below:

**Table 1**

| | **Cy3_staples column 1** |
|---|---|
| 184 | CATAAAGGGAAATAGCAATAGCTAATCAGAGA |

(continued)

| | Cy3_staples column 1 |
|---|---|
| 185 | ACCAGTAGTTATTTTGTCACAATCAATACATA |
| 186 | GCCGCCACCATCGATAGCAGCACCGCAAAATC |
| 187 | AGTGTACTCACCAGAACCACCACCCCCTCAGA |
| 188 | CCGTATAAACAGTTAAGATACAGG |
| | **Cy3_staples column 2** |
| 189 | CAGCTACAATTTTATCCTGAATCAATCCAAA |
| 190 | TAAGAAAGTAATTGAGCGCTAATTCTTACCG |
| 191 | AAGCCCTTTTAGCAAACGTAGAAAATAGAAA |
| 192 | ATTCATATTGGGAATTAGAGCCAGTAATCAG |
| 193 | TAGCGACACCACCGGAACCGCCTAGAGCCGC |
| | **Cy3_staples column 3** |
| 194 | GCAGTATGTTTAAGAAAAGTAAGGAACAAAG |
| 195 | GAGCCATTGGTTTACCAGCGCCACTTATTAC |
| 196 | CAGAGCCAGAATCAAGTTTGCCTACCGACTT |
| 197 | AAGCGTCATTGACAGGAGGTTGAACCGGAAC |
| 198 | GCCTATTTCGGAACCTATTATTCGTTCCAGT |
| | **Cy3_staples column 4** |
| 199 | CAAGATTAGTTGCTATGTCAAAAA |
| 200 | TGAAAATAATTAACTGAACACCCTCAGATAGC |
| 201 | CGAACAAAGGCATGATTAAGACTCAAGACAAA |
| 202 | AGGGCGACGTGAATTATCACCGTCTTAGCGTC |
| 203 | AGACTGTATTTCATAATCAAAATCGGCAGGTC |
| | **Cy3_staples column 5 (FRET) or Cy5_staples column 4 (quenching)** |
| 204 | ATTTCAACAGTGAATAAGGCTTGCGGCGCAGA |
| 205 | CGGTCAATTCGCCTGATAAATTGTGACTTTTT |
| 206 | CATGAGGAAGCGAAAGACAGCATCGTATCGGT |
| 207 | TTATCAGCAATAATTTTTTCACGTCTGTAGCA |
| 208 | TTCCACAGCCAATAGGAACCCATGGCTCAGTA |
| | **Cy3_staples column 6 (FRET) or Cy5_staples column 3 (quenching)** |
| 209 | TTGTATCACATAAGGGAACCGAACACAAAGCT |
| 210 | CCCTCAGCAGTTTCCATTAAACGGACGGAGAT |
| 211 | TTGCGAATTTGCTTTCGAGGTGAAGATCGTCA |
| 212 | TAGCAAGCACAGCCCTCATAGTTATAAAGGAA |
| 213 | CCAGGCGGATAAGTGCCGTCGAGATTCAGGGA |
| | **Cy3_staples column 7 (FRET) or Cy5_staples column 2 (quenching)** |
| 214 | TGCGATTTACCCAAATCAACGTATGACCAA |
| 215 | CTTTGAAAGCGAAACAAAGTACAGTAAAAT |
| 216 | ACGTAATGAGGCCGCTTTTGCGGTTTCTTA |
| 217 | AACAGCTTATAGAAAGGAACAACGCGTAAC |

(continued)

| | Cy3_staples column 7 (FRET) or Cy5_staples column 2 (quenching) |
|---|---|
| 218 | GATCTAAAGCCACCACCCTCATTGGGTTGA |
| | **Cy3_staples column 8 (FRET) or Cy5_staples column 1 (quenching)** |
| 219 | TACCAAGCGAGGACAGATGAACGGGAACCGGA |
| 220 | GGAGTTAACCACTACGAAGGCACCAGCGATTA |
| 221 | GAGTGAGAGATACCGATAGTTGCGGCTTGCAG |
| 222 | CCCTCAGAGTTTTGTCGTCTTTCCTTTCAGCG |
| 223 | TATAAGTATAGCCCGGAATAGGTGAACCGCCA |
| | **Cy5_staples column 1 (FRET) or bh2_staples column 1 (quenching)** |
| 224 | ATATTACCAATCAGAGACAGGAGGCCGATTAA |
| 225 | TTCAGGTTCGAACGTTAAAGTTTGAGTAACAT |
| 226 | CTTAGGTTTAATTACATCATTTGAATTACCTT |
| 227 | TCAATATCACGTGGCATTTTGAATGGCTATTA |
| 228 | TCAGCTAAAATCATAAAAGCCTGTTTAGTATC |
| | **Cy5_staples column 2 (FRET) or bh2_staples column 2 (quenching)** |
| 229 | TTGCTTTGAAATACCTTAGTAATAACATCA |
| 230 | AACAGAGATTGAGGAGCTGAGAGCCAGCAG |
| 231 | TTTACAAATTTACATGGAAGGGTTAGAACC |
| 232 | CAAAAGAAAATCCAAACGCTGAGAAGAGTC |
| 233 | CGTGTGATAGTCCTGATAAGAGAATATAAA |
| | **Cy5_staples column 3 (FRET) or bh2_staples column 3 (quenching)** |
| 234 | GCTCATGGACGAGCAAACGGTACGCCAGAA |
| 235 | GAAAGGAATAGAACCCTGATAGCCCTAAAA |
| 236 | CAGTACCTCAATTCGAAAGAAACCACCAGA |
| 237 | CTGATGCAGATGATGACATAAATCAATATA |
| 238 | AATAGATAAAATAAGTTCTTACCAGTATAA |
| | **Cy5_staples column 4 (FRET) or bh2_staples column 4 (quenching)** |
| 239 | CTTAATGCAAATGGATGCAAATTAACCGTTGT |
| 240 | ACACGACCGCACTAACGTCAGTATTAACACCG |
| 241 | ATAATACACTGATTGCTCCTGATTGTTTGGAT |
| 242 | CAGAGGCGACTTTTTCTCCTTGAAAACATAGC |
| 243 | CTGACCTATAATTTACGTAATTTAGGCAGAGG |
| | **Cy5_staples column 5 (FRET) or bb650q_staples column 4 (quenching)** |
| 244 | GGCGCCAGGGTGGTTTATCCCTTATAAATCAA |
| 245 | ACTGCCCGCCTTACACCGGCGGGCCGTTTTCA |
| 246 | ACTCAATCGCTCTCACAAAGTTAAACGATGCT |
| 247 | TAACCTCAAGCGAGTATCGCACTCCAGCCAGC |
| 248 | GTTAAATCGCGGGAGATCAACCGTTCTAGCTG |
| | **Cy5_staples column 6 (FRET) or bb650q_staples column 3 (quenching)** |
| 249 | TAAACATCCTTTCCAGGATCCCCGGGTACCGA |

(continued)

| | | Cy5_staples column 6 (FRET) or bb650q_staples column 3 (quenching) |
|---|---|---|
| | 250 | AAGGGATACGCCGGGCTCATAACGGAACGTGC |
| | 251 | TAAATGTGCCGGAAACGGAAGGGCGATCGGTG |
| | 252 | ATACTTTTAGCTCATTTCTGGAGCAAACAAGA |
| | 253 | ATTAAGAGAAGAATTAGTTGATTCCCAATTCT |
| | | Cy5_staples column 7 (FRET) or bb650q_staples column 2 (quenching) |
| | 254 | GACGGGCAACAGCTGCCTGTTTGATGGTGGT |
| | 255 | TGAGCTAACGGCATCACTGTGCACTCTGTGG |
| | 256 | GCATCAGCGGATCAAAGCCGCACAGGCGGCC |
| | 257 | CTTTCAGAGAACAAACGGGGACGACGACAGT |
| | 258 | AATATTTTAAAAATTTGGAGACAGTCAAATC |
| | | Cy5_staples column 8 (FRET) or bb650q_staples column 1 (quenching) |
| | 259 | ATCGTTAACTCACATTATGGTCATAGCTGTT |
| | 260 | GAAACAGCGGGGTCATCAGCGTGGTGCTGGT |
| | 261 | CTCCGTGGGGTGGAGTTACGCCAGCTGGCGA |
| | 262 | GCAAGGATGTTAAAAAATCGTAAAACTAGCA |
| | 263 | CGCGTTTTTTAACATTAGTTTGACCATTAGA |

In one embodiment, the staple strands comprise a polynucleotide sequence that is at least 80%, at least 90%, at least 95%, or at least 99% identical to any of SEQ ID NO: 264 to 295. These staple strands form the locks and include the fastening domains. Their target oligonucleotides can be seen from Table 1 below:

**Table 2**

| | Sequence | Target |
|---|---|---|
| 264 | AGCCCCCGATTTAGAGCTTTT**GATCACTTTTGTGACTATGCAA** | Lock for ODN-153 |
| 265 | CTATCGGCCTTGCTGGTAATT**GATCACTTTTGTGACTATGCAA** | Lock for ODN-153 |
| 266 | GGTCTGAGAGACTACCTTTTT**GATCACTTTTGTGACTATGCAA** | Lock for ODN-153 |
| 267 | CTGTCCAGACGACGACAATTT**GATCACTTTTGTGACTATGCAA** | Lock for ODN-153 |
| 268 | **AGTCACAAAAGTGATC**TGCCAGTTACAAAATAAACAGACAAGAAT | Lock for ODN-153 |
| 269 | **AGTCACAAAAGTGATC**ATAATAAGAGCAAGAAACAATTGGCAACA | Lock for ODN-153 |
| 270 | **AGTCACAAAAGTGATC**AGCCACCACCCTCAGAGCCGCGGTAATAAGTTTTAAC | Lock for ODN-153 |
| 271 | **AGTCACAAAAGTGATC**CAGTGCCTTGAGTAACAGTGC | Lock for ODN-153 |
| 272 | CACAATTCCACACAACATA**ACGGGTGCGATTTCTGTGTGAGA** | Lock for ODN-342 |
| 273 | GCCAACGGCAGCACCGTCG**ACGGGTGCGATTTCTGTGTGAGA** | Lock for ODN-342 |
| 274 | ATAATCAGAAAAGCCCCAA**ACGGGTGCGATTTCTGTGTGAGA** | Lock for ODN-342 |
| 275 | CTGTTTAGCTATATTTTCA**ACGGGTGCGATTTCTGTGTGAGA** | Lock for ODN-342 |
| 276 | **ACAGAAATCGCACCCG**TTTTTTTTTGACCTTCATCAAGAGTAAT | Lock for ODN-342 |
| 277 | **ACAGAAATCGCACCCG**TTTTTTTTTACACTAAAACACTCATCTT | Lock for ODN-342 |
| 278 | **ACAGAAATCGCACCCG**TTTTTTTTTGGGATTTTGCTAAACAACT | Lock for ODN-342 |
| 279 | **ACAGAAATCGCACCCG**TTTTTTTTTCGCCACCCTCAGAACCGCC | Lock for ODN-342 |
| 280 | AGCCCCCGATTTAGAGCTTTTT**CAACATCAGTCTGATAAGCTA** | Lock for miR-21 |

(continued)

| | Sequence | Target |
|---|---|---|
| 281 | CTATCGGCCTTGCTGGTAATTT**CAACATCAGTCTGATAAGCTA** | Lock for miR-21 |
| 282 | GGTCTGAGAGACTACCTTTTTT**CAACATCAGTCTGATAAGCTA** | Lock for miR-21 |
| 283 | CTGTCCAGACGACGACAATTTT**CAACATCAGTCTGATAAGCTA** | Lock for |
| | | miR-21 |
| 284 | **ATCAGACTGATGTTGA**TGCCAGTTACAAAATAAACAGACAAGAATTGAGTTAA | Lock for miR-21 |
| 285 | **ATCAGACTGATGTTGA**ATAATAAGAGCAAGAAACAATTGGCAACA | Lock for miR-21 |
| 286 | **ATCAGACTGATGTTGA**AGCCACCACCCTCAGAGCCGCGGTAATAAGTTTTAAC | Lock for miR-21 |
| 287 | **ATCAGACTGATGTTGA**CAGTGCCTTGAGTAACAGTGC | Lock for miR-21 |
| 288 | CACAATTCCACACAACATA**AACTATACAACCTACTACCTCA** | Lock for let-7a |
| 289 | GCCAACGGCAGCACCGTCG**AACTATACAACCTACTACCTCA** | Lock for let-7a |
| 290 | ATAATCAGAAAAGCCCCAA**AACTATACAACCTACTACCTCA** | Lock for let-7a |
| 291 | CTGTTTAGCTATATTTTCA**AACTATACAACCTACTACCTCA** | Lock for let-7a |
| 292 | **AGTAGGTTGTATAGTT**TTTTTTTTGACCTTCATCAAGAGTAAT | Lock for let-7a |
| 293 | **AGTAGGTTGTATAGTT**TTTTTTTACACTAAAACACTCATCTT | Lock for let-7a |
| 294 | **AGTAGGTTGTATAGTT**TTTTTTTGGGATTTTGCTAAACAACT | Lock for let-7a |
| 295 | **AGTAGGTTGTATAGTT**TTTTTTTCGCCACCCTCAGAACCGCC | Lock for let-7a |

In these sequences, the fastening domains are marked in bold print. According to the invention, these fastening domains can be replaced according to the target oligonucleotide.

In a preferred embodiment, the biosensor comprises a scaffold strand that is least 90% identical to SEQ ID NO: 1 and staple strands that are at least 90% identical to SEQ ID NO 1: 295.

The biosensor according to the invention is capable of detecting at least two target oligonucleotides. The target oligonucleotide may be any single or double stranded oligonucleotide of interest. In one embodiment, the target oligonucleotides are selected from group consisting of miRNA, mRNA, lncRNA, rRNA, DNA and ctDNA.

In a preferred embodiment, the target oligonucleotides are associated with a medical condition or disease, i.e. the target oligonucleotides are biomarkers. Thus, the biosensor according to the invention is useful for diagnosing a disease, in particular a disease associated with altered miRNA expression. Diseases that can be diagnosed with the help of the biosensor according to the invention include cardiovascular diseases, inflammatory diseases autoimmune diseases, neurodegenerative diseases, metabolic conditions, renal diseases, pulmonary diseases, infectious diseases and cancer.

In one embodiment, the target oligonucleotides are selected from group consisting of let-7a, miR-139-5p, miR-191, miR-223-3p, miR-376c, let-7b-3p, miR-14, 1-3p miR-192-3p, miR-223-5p, miR-376c-3p, let-7b-5p, miR-141-5p, miR-192-5p, miR-23a, miR-378, let-7c-3p, miR-142-5p, miR-198, miR-27a-3p, miR-423-5p, let-7g,miR-146b-3p miR-199a-3p, miR-27a-5p, miR-4257-3p, miR-1, miR-148a, miR-199a-5p, miR-28-3p, miR-451, miR-100,miR-148b, miR-19b-3p, miR-296-5p, miF 301a, miR-625, miR-107, miR-15b, miR-205-3p,miR-30a, miR-7, miR-10a, miR-16, miR-205-5p, miR-30a-3p, miR-320b, miR-801, miR-1246, miR-181-5p, miR-210-3p, miR-34, miR-885-5p, miR-125a-3p, miR-181a-5p, miR-214, miR-342-5p, miR-92a, miR-126-3p, miR-181b, miR-215, miR-34a, miR-92b, miR-127-3p, miR-181c, miR-216, miR-361-3p, miR-93, miR-1290, miR-182-5p, miR-218

All of these miRNAs are related to cancer and thus, their detection is useful in the context of diagnosing cancer. Since the biosensor according to the invention may detect two or more target oligonucleotides at the same time, it is particularly useful for a reliable detection of cancer. Thus, the biosensor according to the invention is for use in diagnosing cancer.

In another aspect, the invention is directed to a method for detecting at least two target oligonucleotides in a sample, the method comprising

a) bringing a biosensor according to any of claims 1 to 9 into contact with the sample.

The sample may be any biological sample that may contain oligonucleotides, for example tissue, cells or parts thereof, cell culture supernatant, blood, serum or other bodily fluids.

In one embodiment, in the method according to the invention, a secondary probe is added to the sample after step a), the secondary probe being capable of hybridizing to the first or second fastening domain to which the toehold domain is not attached. The first or second domain to which the toehold domain is not attached is not capable of hybridizing to the target nucleotide. Consequently, said domain is free when the other domain is hybridized to the target nucleotide and is capable of hybridizing with the secondary probe. The secondary probe is not capable of hybridizing with either fastening domain while the fastening domains are hybridized to each other and is capable of hybridizing only with a free fastening domain. The secondary probe may comprise a moiety for signal amplification, e.g., a fluorescent, radioactive, luminescent or chromogenic moiety, nanoparticles or gold or silvers particles allowing detection via SERS.

In another embodiment, during step a), a molecular crowder is added to the sample. A molecular crowder is an inert polymer capable of modulating folding, assembly, structures and interactions of macromolecules such as oligonucleotides through physical effects, including volume exclusion and dehydration, without a direct interaction with the macromolecules. Examples of molecular crowders include polyethylene glycol (PEG), diethylene glycol (DEG) and polyvinylpyrrolidone (PVP). Adding a molecular crowder to the sample is advantageous since it enhance the rate of hybridization of DNA molecules and strand exchange kinetics, thereby facilitating displacement of the fastening domain by the target oligonucleotides. This increases the sensitivity of DNA origami biosensor.

In yet another aspect, the invention is directed to a method for diagnosing cancer, the method using the biosensor according to the invention.

## Examples

### Example 1

*Design and assembly of DNA origami book biosensor*

The structure was designed using caDNAno software (http://cadnano.org/) (S. M. Douglas, A. H. Marblestone, S. Teerapittayanon, A. Vazquez, G. M. Church and W. M. Shih, Nucleic Acids Res, 2009, 37, 5001-5006). The generated file from caDNAno software was submitted to CanDo (Computer-aided engineering for DNA origami, http://cando-dna-or igami.org/) for further evaluation of the shape, flexibility, and dynamics. The assembly of the DNA origami book biosensor is achieved by mixing 10 nM single-stranded scaffold DNA (Type: p8064, Europhins MWG Operon, Ebersberg, Germany) with unmodified staple strands (100 nM each) (HPSF purified, LubioScience-Switzerland IDT, Zurich, Switzerland) and 1 $\mu$M of modified staple strands with fluorophores (ddUTP-Cy3 and ddUTP-Cy5, Jena Bioscience, Germany), oligonucleotides with bh2 and bbq650q (Biomers, Ulm, Germany) and 1 $\mu$M of biotin modified oligonucleotides (Biomers, Ulm, Germany). All modified oligonucleotides were purified by HPLC. The sequences of all oligonucleotides used are given in Tables 1, 2 and 3. The annealing of the scaffold DNA and staple strands mix was done by using the temperature ramp method: heating the mix solution to 80 °C for 5 min, cooling to 65 °C during the first 15 min, and cooling further down to 4 °C for 16 hrs.

SEQ ID NO: 1:

tgatagacggttttttcgcccttttgacgttggagtccacgttcttttaatagtggactcttgttccaaactggaacaacactcaacccctatctcgggct

attctttttgatttataaggggattttgccgatttcggaaccaccatcaaacaggattttcgcctgctggggcaaaccagcgtggaccgcttgctgc

aactctctcagggccaggcggtgaagggcaatcagctgttgcccgtctcactggtgaaaagaaaaaccaccctggcgcccaatacgca

aaccgcctctcccgcgcgttggccgattcattaatgcagctggcacgacaggtttcccgactggaaagcgggcagtgagcgcaacgca

attaatgtgagttagctcactcattaggcaccccaggctttacactttatgcttccggctcgtatgttgtgtggaattgtgagcggataacaatttc

acacaggaaacagctatgaccatgattacgaattcgagctcggtacccggggatcctcaactgtgaggaggctcacggacgcgaagaa

caggcacgcgtgctggcagaaaccccccggtatgaccgtgaaaacggcccgccgcattctggccgcagcaccacagagtgcacaggc

gcgcagtgacactgcgctggatcgtctgatgcagggggcaccggcaccgctggctgcaggtaacccggcatctgatgccgttaacgattt

gctgaacacaccagtgtaagggggatgtttatgacgagcaaagaaacctttacccattaccagccgcagggcaacagtgacccggctcata

ccgcaaccgcgcccggcggattgagtgcgaaagcgcctgcaatgaccccgctgatgctggacacctccagccgtaagctggttgcgtgg

gatggcaccaccgacggtgctgccgttggcattcttgcggttgctgctgaccagaccagcaccacgctgacgttctacaagtccggcacgtt

ccgttatgaggatgtgctctggccggaggctgccagcgacgagacgaaaaaacggaccgcgtttgccggaacggcaatcagcatcgttt

aactttacccttcatcactaaaggccgcctgtgcggcttttttttacgggattttttttatgtcgatgtacacaaccgcccaactgctggcggcaaat

gagcagaaatttaagtttgatccgctgtttctgcgtctcttttttccgtgagagctatcccttcaccacggagaaagtctatctctcacaaattccgg

gactggtaaacatggcgctgtacgtttcgccgattgtttccggtgaggttatccgttcccgtggcggctccacctctgaaagcttggcactggc

cgtcgttttacaacgtcgtgactgggaaaaccctggcgttacccaacttaatcgccttgcagcacatccccctttcgccagctggcgtaatag

cgaagaggcccgcaccgatcgcccttcccaacagttgcgcagcctgaatggcgaatggcgctttgcctggtttccggcaccagaagcggt

gccggaaagctggctggagtgcgatcttcctgaggccgatactgtcgtcgtcccctcaaactggcagatgcacggttacgatgcgcccatc

tacaccaacgtgacctatcccattacggtcaatccgccgtttgttcccacggagaatccgacgggttgttactcgctcacatttaatgttgatga

aagctggctacaggaaggccagacgcgaattattttttgatggcgttcctattggttaaaaaatgagctgatttaacaaaaatttaatgcgaatt

ttaacaaaatattaacgtttacaatttaaatatttgcttatacaatcttcctgttttttggggctttttctgattatcaaccggggtacatatgattgacatg

ctagtttttacgattaccgttcatcgattctcttgtttgctccagactctcaggcaatgacctgatagcctttgtagatctctcaaaaatagctaccct

ctccggcattaatttatcagctagaacggttgaatatcatattgatggtgatttgactgtctccggcctttctcacccttttgaatctttacctacaca

ttactcaggcattgcatttaaaatatatggggttctaaaaattttttatccttgcgttgaaataaaggcttctcccgcaaaagtattacagggtcata

atgttttttggtacaaccgatttagctttatgctctgaggctttattgcttaatttttgctaattctttgccttgcctgtatgatttattggatgttaatgctacta

ctattagtagaattgatgccacctttttcagctcgcgccccaaatgaaaatatagctaaacaggttattgaccatttgcgaaatgtatctaatggt

caaactaaatctactcgttcgcagaattgggaatcaactgttatatggaatgaaacttccagacaccgtactttagttgcatatttaaaacatgt

tgagctacagcattatattcagcaattaagctctaagccatccgcaaaaatgacctcttatcaaaaggagcaattaaaggtactctctaatcc

tgacctgttggagtttgcttccggtctggttcgctttgaagctcgaattaaaacgcgatatttgaagtctttcgggcttcctcttaatctttttgatgca

atccgctttgcttctgactataatagtcagggtaaagacctgattttttgatttatggtcattctcgttttctgaactgtttaaagcatttgagggggatt

caatgaatatttatgacgattccgcagtattggacgctatccagtctaaacattttactattaccccctctggcaaaacttcttttgcaaaagcctc

tcgctattttggtttttatcgtcgtctggtaaacgagggttatgatagtgttgctcttactatgcctcgtaattccttttggcgttatgtatctgcattagtt

gaatgtggtattcctaaatctcaactgatgaatctttctacctgtaataatgttgttccgttagttcgttttattaacgtagattttttcttcccaacgtcct

gactggtataatgagccagttcttaaaatcgcataaggtaattcacaatgattaaagttgaaattaaaccatctcaagcccaatttactactcg

ttctggtgtttctcgtcagggcaagccttattcactgaatgagcagctttgttacgttgatttgggtaatgaatatccggttcttgtcaagattactctt
gatgaaggtcagccagcctatgcgcctggtctgtacaccgttcatctgtcctctttcaaagttggtcagttcggttccctttatgattgaccgtctgc
gcctcgttccggctaagtaacatggagcaggtcgcggatttcgacacaatttatcaggcgatgatacaaatctccgttgtactttgtttcgcgct
tggtataatcgctggggggtcaaagatgagtgttttagtgtattctttttgcctctttcgtttttaggttggtgccttcgtagtggcattacgtattttacccgt
ttaatggaaacttcctcatgaaaaagtctttagtcctcaaagcctctgtagccgttgctaccctcgttccgatgctgtcttcgctgctgagggtga
cgatcccgcaaaagcggcctttaactccctgcaagcctcagcgaccgaatatatcggttatgcgtgggcgatggttgttgtcattgtcggcgc
aactatcggtatcaagctgtttaagaaattcacctcgaaagcaagctgataaaccgatacaattaaaggctccttttggagccttttttttggag
attttcaacgtgaaaaaattattattcgcaattcctttagttgttcctttctattctcactccgctgaaactgttgaaagttgtttagcaaaatcccata
cagaaaattcatttactaacgtctggaaagacgacaaaactttagatcgttacgctaactatgagggctgtctgtggaatgctacaggcgttg
tagtttgtactggtgacgaaactcagtgttacggtacatgggttcctattgggcttgctatccctgaaaatgagggtggtggctctgagggtggc
ggttctgagggtggcggttctgagggtggcggtactaaacctcctgagtacggtgatacaccattccgggctatacttatatcaaccctctcg
acggcacttatccgcctggtactgagcaaaaccccgctaatcctaatccttctcttgaggagtctcagcctcttaatactttcatgtttcagaata
ataggttccgaaataggcaggggggcattaactgtttatacgggcactgttactcaaggcactgacccccgttaaaacttattaccagtacactc
ctgtatcatcaaaagccatgtatgacgcttactggaacggtaaattcagagactgcgctttccattctggctttaatgaggatttatttgtttgtga
atatcaaggccaatcgtctgacctgcctcaacctcctgtcaatgctggcggcggctctggtggtggttctggtggcggctctgagggtggtgg
ctctgagggtggcggttctgagggtggcggctctgagggaggcggttccggtggtggctctggttccggtgattttgattatgaaaagatggc
aaacgctaataaggggctatgaccgaaaatgccgatgaaaacgcgctacagtctgacgctaaaggcaaacttgattctgtcgctactga
ttacggtgctgctatcgatggtttcattggtgacgtttccggccttgctaatggtaatggtgctactggtgattttgctggctctaattcccaaatggc
tcaagtcggtgacggtgataattcacctttaatgaataaattccgtcaatatttaccttccctccctcaatcggttgaatgtcgcccttttgtctttggc
gctggtaaaccatatgaattttctattgattgtgacaaaataaacttattccgtggtgtctttgcgtttctttatatgttgccacctttatgtatgtattttc
tacgtttgctaacatactgcgtaataaggagtcttaatcatgccagttcttttgggtattccgttattattgcgtttcctcggtttccttctggtaactttgt
tcggctatctgcttacttttcttaaaaagggcttcggtaagatagctattgctatttcattgtttcttgctcttattattgggcttaactcaattcttgtggg
ttatctctctgatattagcgctcaattaccctctgactttgttcagggtgttcagttaattctcccgtctaatgcgcttccctgtttttatgttattctctctgt
aaaggctgctattttcatttttgacgttaaacaaaaaatcgtttcttatttggattgggataaataatatggctgtttattttgtaactggcaaattagg
ctctggaaagacgctcgttagcgttggtaagattcaggataaaattgtagctgggtgcaaaatagcaactaatcttgatttaaggcttcaaaa
cctcccgcaagtcgggaggttcgctaaaacgcctcgcgttcttagaataccggataagccttctatatctgatttgcttgctattgggcgcggta
atgattcctacgatgaaaataaaaacggcttgcttgttctcgatgagtgcggtacttggtttaatacccgttcttggaatgataaggaaagaca
gccgattattgattggtttctacatgctcgtaaattaggatgggatattattttttcttgttcaggacttatctattgttgataaacaggcgcgttctgcat
tagctgaacatgttgtttattgtcgtcgtctggacagaattactttaccttttgtcggtactttatattctcttattactggctcgaaaatgcctctgccta
aattacatgttggcgttgttaaatatggcgattctcaattaagccctactgttgagcgttggctttatactggtaagaatttgtataacgcatatgat
actaaacaggctttttctagtaattatgattccggtgtttattcttatttaacgccttatttatcacacggtcggtatttcaaaccattaaatttaggtca
gaagatgaaattaactaaaatatatttgaaaaagttttctcgcgttctttgtcttgcgattggatttgcatcagcatttacatatagttatataaccc
aacctaagccggaggttaaaaaggtagtctctcagacctatgattttgataaattcactattgactcttctcagcgtcttaatctaagctatcgct
atgttttcaaggattctaagggaaaattaattaatagcgacgatttacagaagcaaggttattcactcacatatattgatttatgtactgtttccatt
aaaaaaggtaattcaaatgaaattgttaaatgtaattaattttgttttcttgatgtttgtttcatcatcttctttttgctcaggtaattgaaatgaataattc
gcctctgcgcgattttgtaacttggtattcaaagcaatcaggcgaatccgttattgtttctcccgatgtaaaaggtactgttactgtatattcatctg
acgttaaacctgaaaatctacgcaatttctttatttctgttttacgtgcaaataattttgatatggtaggttctaacccttccattattcagaagtataa
tccaaacaatcaggattatattgatgaattgccatcatctgataatcaggaatatgatgataattccgcrccttcrggtggtttctttgttccgcaa
aatgataatgttactcaaacttttaaaattaataacgttcgggcaaaggatttaatacgagttgtcgaattgtttgtaaagtctaatacttctaaat

cctcaaatgtattatctattgacggctctaatctattagttgttagtgctcctaaagatatttttagataaccttcctcaattcctttcaactgttgatttgccaactgaccagatattgattgagggtttgatatttgaggttcagcaaggtgatgctttagattttttcatttgctgctggctctcagcgtggcactgttgcaggcggtgttaatactgaccgcctcacctctgttttatcttctgctggtggttcgttcggtattttaatggcgatgtttagggctatcagttcgcgcattaaagactaatagccattcaaaaatattgtctgtgccacgtattcttacgctttcaggtcagaagggttctatctctgttggccagaatgtcccttttattactggtcgtgtgactggtgaatctgccaatgtaaataatccatttcagacgattgagcgtcaaaatgtaggtatttccatgagcgttttcctgttgcaatggctggcggtaatattgttctggatattaccagcaaggccgatagtttgagttcttctactcaggcaagtgatgttattactaatcaaagaagtattgctacaacggttaatttgcgtgatggacagactctttttactcggtggcctcactgattataaaaacacttctcaggattctggcgtaccgttcctgtctaaaatccctttaatcggcctcctgtttagctcccgctctgattcaacgaggaaagcacgttatacgtgctcgtcaaagcaaccatagtacgcgccctgtagcggcgcattaagcgcggcgggtgtggtggttacgcgcagcgtgaccgctacacttgccagcgcctagcgcccgctcctttcgctttcttcccttcctttctcgccacgttcgccggctttccccgtcaagctctaaatcggggggctccctttagggttccgatttagtgctttacggcacctcgaccccaaaaaacttgatttgggtgatggttcacgtagtgggccatcgccc

**Table 3**

| 2 | GTCTATCATCGGAACCGAAAGGAAGTGCTTTC | Core staple |
|---|---|---|
| 3 | CGTGGACTCCAACGTCCGCTAGGGTACTATGG | Core staple |
| 4 | GAACAAGATGCGCGTACCGCCGCG | Core staple |
| 5 | AAGAATAGCCCGAGATGGCGGTTTCCTGTCGT | Core staple |
| 6 | TCCGAAATCGGCAAATTCTTTTCTTGCGCTC | Core staple |
| 7 | CCCAGCAGGCGAAAATATTGCCCTCTAATGAG | Core staple |
| 8 | TGGCCCTGAGAGAGTTGTCCACGCTGGTTTGC | Core staple |
| 9 | GCCAGCTGGGGTAAAGTTCTGCCA | Core staple |
| 10 | CATAAAGTGGTGCCGGATCAGACGATCCAGCG | Core staple |
| 11 | AGGGATTTAGTAGAAGTTGCAACACTGAAAGC | Core staple |
| 12 | TCCTGAGATTCTTTGATACATTTTTTCTGGCC | Core staple |
| 13 | ACCGAGTATCCATCACTATTTACACACCAGTC | Core staple |
| 14 | CCTCCTCACCGGGGGGTGTTTCTTTGGTATGAG | Core staple |
| 15 | GCTCGAATCCAGAATGTGGTGTGTGCTTTCGC | Core staple |
| 16 | TCCTGTGTCTGCGCGCGATGCCGGGGTGTCCA | Core staple |
| 17 | CTTGCCTGTAGACAGGCGTATAACGGGAAGAAAGCGAAA | Core staple |
| 18 | AGCAATACAGTGTTTTAGGGCGCGCGCTGGCA | Core staple |
| 19 | CGGTCATACAGTTGAGTCGGGAAAGCGTATTG | Core staple |
| 20 | TGCTGCGGTCGTAATCAATTGCGACCAGTGA | Core staple |
| 21 | CAGTGTCAGAAATTGTTGGGGTGCTCACCGCC | Core staple |
| 22 | ATCGTCTGGCCGCTACTATAATCAGTGAGGCC | Core staple |
| 23 | GTAAGAATTGGTCAGTAGCATCACCTTGCTGA | Core staple |
| 24 | CCGGGTCAGAGAGATAAACGCGGT | Core staple |
| 25 | CACGCAACAGCAGTTGTACATCGACATAAAA | Core staple |
| 26 | GTCTTTAAAAATCTAATGGCAAATTATTAAAT | Core staple |
| 27 | CATCGCCAAGTGCCACAGGTTATCTATTAGAC | Core staple |
| 28 | GCAGAAGAGTGAGGCGAACTAATAGTCAATAG | Core staple |

(continued)

| 29 | CGGCCAGATTCCGGCAGACTTTCTAAACGTAC | Core staple |
|---|---|---|
| 30 | CGGACTTGTGAAGGGTGGAAAAAGGGAACGGA | Core staple |
| 31 | CTGGTCAGTAAAAAAACTTAAATTGTGCCAAG | Core staple |
| 32 | CAAATGAATGCGCGAACTTCTGACGGAAAAAC | Core staple |
| 33 | CCTGCAACTTAAAAATAAGGGACAGACGCTCA | Core staple |
| 34 | GATTGCCGGCACATCCGCGGTTGCGCTCGTCA | Core staple |
| 35 | TTTAGTGATAGAACGTTGCAGGCTCAGCAA | Core staple |
| 36 | AAATCCCGCAGCAACCCGGCTGGAGTTACCTG | Core staple |
| 37 | CTTTAGGAAGTAATAAACCGAACGAACCACCA | Core staple |
| 38 | AGCGCCATCCTGTAGCTGGTGCCG | Core staple |
| 39 | CACGACGTGTCACGTTGGCGCATCGTAACCGT | Core staple |
| 40 | TATCATTTCAAAATTAGATGAATAATCAAGAA | Core staple |
| 41 | AGGAGCGGTGAATAATCGGGAGAATACCTGAG | Core staple |
| 42 | CAGATGATCAATATAATTTGAATAAAATCGCG | Core staple |
| 43 | GGCTGCGCACCGCTTCCAGCTTTCAATAGGAA | Core staple |
| 44 | CGGGCCTCCAGGAAGAACAACCCGAAATTTTT | Core staple |
| 45 | AAGGGGGACAGTTTGAGGCGGATTTAAACGTT | Core staple |
| 46 | TACCATATTGCGGAACACAACTCGCAACAGTT | Core staple |
| 47 | TATACTTCAATTATCATTTAGAAGTAAAATAT | Core staple |
| 48 | TTTCCGGCAACTGTTGAATCGGCGCCGTGGTG | Core staple |
| 49 | ATCGGCCTTTCGCTACCGCCACGAGACGCA | Core staple |
| 50 | GCATCTGCTGTGCTGCGACGGCCATCTGCTCA | Core staple |
| 51 | GGATTCGCTTTGAGGATCATATTCCTGATTAT | Core staple |
| 52 | CTGGCCTTGTTTACCACGCCATTCGCCATTCA | Core staple |
| 53 | TGGGATAGTGTAAAACAAGGCGATTAAGTTGG | Core staple |
| 54 | CGCCATCATACCAAAAGAGGGTAG | Core staple |
| 55 | TTGTATAAGCAATGCCGTAGGTAAAGATTCAA | Core staple |
| 56 | TTTTAATGATTTATCATAACTATATAAGAATA | Core staple |
| 57 | TGTGAGTGAGATTAAGTCGCAAGAATACCGAC | Core staple |
| 58 | CGCTATTACCTTAGAAAAATATATTTCATCTT | Core staple |
| 59 | GGCTATCAGGTCATTGATGCCGGAACATTATGAAGCAATA | Core staple |
| 60 | GAATCGATTATGATATAGCCTTTATCATACAG | Core staple |
| 61 | TGTCAATCGAAAGGCCTTAGAACCACTAATAG | Core staple |
| 62 | AATAGTGAGAAACAGTAAACAAACTACAGTAA | Core staple |
| 63 | GATAGCTTAATAACCTATTTCAATACAATAAC | Core staple |
| 64 | ATAAATTACCTGAGAGTTTTAACCATCAACAT | Core staple |
| 65 | ACCATCAAGAACGGTTTCGCATTTCGGATT | Core staple |
| 66 | AAGGGTGAATATGTACTTAAATTGGACCGTAA | Core staple |
| 67 | GCGAGAAAAATTATTCTGCTTCTGTAAATCGT | Core staple |
| 68 | AAGCCTCAAGCAAAGCTGTTTTAA | Core staple |

(continued)

| 69 | GCTGAAACAAACTCCGATTAGAGAGTACCTT | Core staple |
|---|---|---|
| 70 | ATATGCGTAAAAGGTAGCGCCTGTCGAGAACA | Core staple |
| 71 | AGCCAACGAGCCAGTAAACAAGAAGAACGGGT | Core staple |
| 72 | ATCGCCATGCCAACATGAGCATGTCAATAATCGGCTGTCT | Core staple |
| 73 | CTGGAAGTTTCATTCCGCTCAACAGGATTGCATCAAAAATCAGGTCTT | Core staple |
| 74 | GCGAACGACTTAGAGCAAAGACTTTAAACAGT | Core staple |
| 75 | TACATTTCCCTTTTGACTTCAAAGTAAATATT | Core staple |
| 76 | GTACCGACTATACAAAGCGTTAAATGTAAATG | Core staple |
| 77 | CATTTTCGCTCAACAGGGTTTGAACAAAGAAC | Core staple |
| 78 | ATGCTGTAATATAACAGCAAAATTACCCTGTA | Core staple |
| 79 | GCGGATGGGTAGATTCCAATAAATTTCAAC | Core staple |
| 80 | TAATTGCTGCAAATGGTCAATTCTCTCATATA | Core staple |
| 81 | TCCCATCCAATTTAATTAGGGCTTAATTGAGA | Core staple |
| 82 | TTCCTTATAACGCGAGGCGTTTTAGCGAACCTCCCGACTT | Core staple |
| 83 | TACCCTGACTACCAATAGAAATTATAGTCAGAGAGCATAATACGGTGT | Core staple |
| 84 | GCGTCCAATACTGCGGTGCCAGAGGGGGTAAT | Core staple |
| 85 | CCAATAGCAAGCAAATGCACTCATTTATCAAC | Core staple |
| 86 | TATCCGGTATTCTAAGCATTCCAAAAATAATA | Core staple |
| 87 | ATTACGAGGCATAGTAAGAGCAACACTATCATACCATAAATCAAAAAG | Core staple |
| 88 | ACGACGATAAAAACCAAAATGCTTCAAATAT | Core staple |
| 89 | TTTGCAAAAGAAGTTTAATCGTCACGAACCAG | Core staple |
| 90 | TTTAGGAATACCACATTCAACTAAATGGTTTA | Core staple |
| 91 | TATTACAGGTAGAAAGATTCATCTACCTTA | Core staple |
| 92 | TAAAACGAACTAACGGATACCAGTCAGGACGT | Core staple |
| 93 | CTCGTTAGGCCAGCCAAACTCAAA | Core staple |
| 94 | ACCGGAAGAGGTGGCATCAATAAC | Core staple |
| 95 | CCTTTGCCTAACGTCATTTGCACGTAAAACAG | Core staple |
| 96 | GATAACCCCCATATTATTTATCCCTTACCAACGCTAACGA | Core staple |
| 97 | TCAGAGGCGATTTTTTGTTTAACTTTGCACC | Core staple |
| 98 | GCTCATTCTTTAATCATTGTGAATAGTTGAGA | Core staple |
| 99 | TATTCATTTAAGAACTGGCTCATTAACAACAT | Core staple |
| 100 | CGCCAGCATACATGGCTTTTGATTGCCCCCT | Core staple |
| 101 | AGACGATTAGTCTCTGAATTTACCTGAAACATGAAAGTAT | Core staple |
| 102 | GGAGCGGGAAAGGGCGAAAAACC | Core staple |
| 103 | AGTGTAGCGGTCACGCGTCCACTATTAAAGAA | Core staple |
| 104 | GCAAGGCAGAAGCCCGTTAATTGCTGAATATA | Core staple |
| 105 | TAGTAGCAAATTCGAGTAAGAGGTCATTTTT | Core staple |
| 106 | AGCAAGCCGTTTTTATGGAATCATTACCGCGC | Core staple |
| 107 | ATTAAACCAAGTACCCAGATATAGAAGGCT | Core staple |
| 108 | TCAGAAAACGAGAATGAACCCTCGTTTACCAG | Core staple |

(continued)

| 109 | CATTGAATCCCCCTCAAATAGCGAGAGGCT | Core staple |
|---|---|---|
| 110 | AACAAAATGGGTTATAAAATCATA | Core staple |
| 111 | CCGGCGAACGTGGCGACTAAAGGG | Core staple |
| 112 | CAGCCAGCGTAAAGCCTATCCGCT | Core staple |
| 113 | TTTGCCGCCCAGCTTAGCAAGAAT | Core staple |
| 114 | TTTTAAATGCAAATATCCCGGTTG | Core staple |
| 115 | AACACCGGTGCAGAACAAGTAATT | Core staple |
| 116 | CTTGACAATGTACAGACCAGGCGC | Core staple |
| 117 | TGACCCCCAACCTAAAACGAAAGA | Core staple |
| 118 | ATATTCGGTCGCTGAGCCGACAATGACAACAA | Core staple |
| 119 | TTCAACAGAGACGTTAGTAAATGA | Core staple |
| 120 | ACCCTCAGTATCACCGTACTCAGG | Core staple |
| 121 | CGGGGAGAAGGGTTGAGTGTTGTTAAGGATTA | Foot staple |
| 122 | AAAGATGTAATCACACACCACCGGCCAACGCG | Foot staple |
| 123 | GCACGCGTAATCACCATTCGTCCT | Foot staple |
| 124 | CTGGTAATCATTAATGTCTTCGCGTCCGTGAG | Foot staple |
| 125 | TAAAATCTCTGAGATTTTGGCCGG | Foot staple |
| 126 | CCGTTTTTTCACTCCAAAAGGTAG | Foot staple |
| 127 | GATTACCGGAATTTGTCTGTTGCCCGTCGCTGGCAGCCTC | Foot staple |
| 128 | GAAACCAGGTAACAGACGGCTAAT | Foot staple |
| 129 | CTATTTTTCGCCATGTTGCTCAAT | Foot staple |
| 130 | TTTAGTAAATCGGTTGAAAATAATTCGTTACACCAAGCGT | Foot staple |
| 131 | ATATGCAATAAGAGTAAGAACAAA | Foot staple |
| 132 | CAAAAGGAGCGGGAGGGAATAACACTAACAACATTTAAAGAGCTTAAT | Foot staple |
| 133 | CAGGGAAGCGAGGAAAGAGTTTTCATTAAAGATCTACAAA | Foot staple |
| 134 | AATAACGGGAGGGAAGGCATTCATGGCAAAAGGTCGAGCC | Foot staple |
| 135 | ATTGACGGATTTTCGGTCGCAGAG | Foot staple |
| 136 | CCCCCTTAAACAAATAGCCGTCTG | Foot staple |
| 137 | CATTAAAGCTCAAGAGCCAGTTTG | Foot staple |
| 138 | TACAGAGATTTTGAAGCCTTAAAT | Hinge staple |
| 139 | ACAACGCTGAAAATCTCCAAAAAAAAGGAGC | Hinge staple |
| 140 | TAAGAGGCTGAGACTCCCAGAATGGAAAGCGCGGCCTTGA | Hinge staple |
| 141 | GGATTAGCGGGGGTTTTTACCGTAACACTGAGTGTACAAACT | Hinge staple |
| 142 | GGCTTGAGTGCAGATACATAACGC | Hinge staple |
| 143 | AAGGAAACCGCATTAGACGGGAGAGCAGCCTT | Hinge staple |
| 144 | CGGAACGACCTGACGAGAAACACCGTAAATTG | Hinge staple |
| 145 | GATTGAGGAATACCCAAAAGAACTGTTACCAG | Hinge staple |
| 146 | GGACTAAAGTCGAAATCCGCGACCTACTTAGC | Hinge staple |
| 147 | TCATCGGCAAATTATTCATTAAAGATTCAACC | Hinge staple |
| 148 | CTTTAATTGGAACGAGGGTAGCAAGGCTTTGA | Hinge staple |

(continued)

| 149 | TATTCACATTAGCGTTTGCCATCTGCGCGTTT | Hinge staple |
|---|---|---|
| 150 | ACCGGAAGAGGTGGCATCAATAAC | Staple with biotin |
| 151 | CTCGTTAGGCCAGCCAAACTCAAA | Staple with biotin |
| 152 | CCTTTGCCTAACGTCATTTGCACGTAAAACAG | Staple with biotin |
| 153 | AATAGATAAAATAAGTTCTTACCAGTATAA | Staple with biotin |
| 154 | TGAGCTAACGGCATCACTGTGCACTCTGTGG | Staple with biotin |
| 155 | CTTTCAGAGAACAAACGGGGACGACGACAGT | Staple with biotin |
| 156 | **CCC**GACGGGGAAAGC | Endcap staple |
| 157 | **CCCC**CGAGCCGGAAG | Endcap staple |
| 158 | **CCC**TATCCAGAACAA | Endcap staple |
| 159 | **CCC**GTGGTGCCATCC | Endcap staple |
| 160 | **CCCC**CAAACCCTCAA | Endcap staple |
| 161 | **CCCC**GTTTTCCCAGT | Endcap staple |
| 162 | GTAACGCCAGG**CCCC** | Endcap staple |
| 163 | AAATAAAGAAA**CCCC** | Endcap staple |
| 164 | **CCCC**TTGCGTAGATT | Endcap staple |
| 165 | **CCCC**AAACAGGAAGA | Endcap staple |
| 166 | **CCC**TTAACCTCCGGC | Endcap staple |
| 167 | **CCC**TTTGGGGCGCGA | Endcap staple |
| 168 | **CCC**AAACAACATGTT | Endcap staple |
| 169 | **CCCC**TAGACTGGATA | Endcap staple |
| 170 | AGTAAAATGTT**CCCC** | Endcap staple |
| 171 | GCGTCTTTCCAGAGCCTAATT**CCC** | Endcap staple |
| 172 | **CCCC**ATCTACGTTAA | Endcap staple |
| 173 | TGGGAAGAAAA**CCCC** | Endcap staple |
| 174 | TGAGTTAAGCCCA**CCC** | Endcap staple |
| 175 | ATAGGCTGGCT**CCCC** | Endcap staple |
| 176 | TATAAAAGAAACG**CCC** | Endcap staple |
| 177 | **CCC**CAAAGACACCACGGAATAAGTCACCATTACCATTAGCAAGGC**CCC** | Endcap staple |
| 178 | GGCAAAAGAAT**CCCC** | Endcap staple |
| 179 | **CCCC**GCATAACCGAT | Endcap staple |
| 180 | **CCC**CGGAAACGTCACCAATGAAACCCTCAGAACCGCCACCCTCAGCCC | Endcap staple |
| 181 | CCATCGCCCAC**CCCC** | Endcap staple |
| 182 | ATTTTCTGTAT**CCCC** | Endcap staple |
| 183 | AGGTTTAGTAC**CCCC** | Endcap staple |

*Labeling of oligonucleotides with Cy3 and Cy5 fluorophores by Terminal Transferase (TdT)*

Unmodified staple strands were purchased at a concentration of 100 μM. Selected ones were labelled with ddUTP-Cy3, and ddUTP-Cy5, using Terminal Transferase (TdT) (Roche, Switzerland). TdT reaction was performed in a total volume of 20 μL consisting of TdT reaction buffer (1x), $CoCl_2$ (5 mM), 100 pmol unmodified staple strands and 500 pmol of modified ddUTP conjugates. The solution was mixed well and incubated with TdT (400 U/reaction) for 30 min at 37 °C. TdT reaction was stopped by heating the mixture at 70 °C for 10 min and the oligonucleotides were precipitated with NaOAc (0.1 M, pH 5.2) and 2.5 volumes of 96% EtOH for 1 h at -20 °C. Precipitation followed by centrifugation for 30 min at 13,000 g resulted in a pellet of the product, which was washed with 70% EtOH, dried and dissolved in water. These labelled oligonucleotides were analyzed on HPLC (Agilent Technologies 1260 Infinity II system) with an Agilent Bio SAX, non-porous 4.6 mm X 250 mm, 5 $\mu$m HPLC column, and then used for self-assembly (Figure 16).

*Agarose gel mobility shift assays*

Assembled DNA origami book biosensors were loaded onto the 1.5% agarose gel containing 11 mM $MgCl_2$ and let migrate for 2 hours at 70 V. To detect the migrated structures, gels were stained with 1x SYBR Gold for 30 min and visualized with BioRad Gel Doc XR+.

*Structure purification*

DNA origami book biosensor structures were assembled with an excess of staple strands and purification of unincorporated staple strands was done with two different methods. The first method was based on 100 kDa Amicon Ultra-0.5 mL centrifugal filters (Millipore, Germany). Amicon filters were pre-wet by filling with annealing buffer (1x TAE/10 mM $Mg^{2+}$) with 500 $\mu$L and centrifuged at 8000 g for 10 min. Then the samples were loaded onto the filter (100 $\mu$L), and completed to 500 $\mu$L with the annealing buffer. The sample was centrifuged at 8000 g for 8 min, and washed 4 times with 400 $\mu$L of annealing buffer. The second method used for purification was agarose gel electrophoresis. The sample containing assembled book biosensor structure (100 $\mu$L) and 6x glycerol (20 $\mu$L) was loaded into 1% agarose gel. The gel was run on 70 V for 1 h and the corresponding band for fluorescently labelled structures was cut out from the gel using a razor blade and extracted from the gel by squeezing with a glass slide.

*AFM imaging*

Freshly cleaved mica was incubated with 10 $\mu$L of annealing buffer containing 2 mM $MgCl_2$ for 1 min and rinsed with $ddH_2O$ before deposition of the purified sample (5-10 $\mu$L of 0.8-1 nM sample) for 2 min. Imaging was performed using an AFM NT-MDT (NTEGRA II). All images were analyzed using Gwyddion analysis software.

*TEM Imaging*

TEM carbon grids (Formvar/carbon, 400 mesh, Cu, TedPella, Inc., USA) were plasma-exposed for 1 min (24 W). A sample (5 $\mu$L) was placed and incubated on the grid for 30 sec and then removed with filter paper. Subsequently, samples were negatively stained with 1% Uranyl acetate aqueous solution (5 $\mu$L) containing 25 mM NaOH for 15 sec and excess stain solution was removed with filter paper. Imaging was performed using a JEM-1011 transmission electron microscope (JEOL) operated at 80 kV.

*Fluorescence measurements of DNA origami book biosensors by spectrophotometry*

A solution containing the self-assembled and purified DNA origami book biosensor structures (45 $\mu$L, 0.5 nM), were loaded into a cuvette with a final volume of 60 $\mu$L (Hellma). Fluorescence measurements was done by using a spectrofluorometer (Horiba, Duetta Fluorescence and Absorbance Spectrometer) and emission spectra of the different book biosensors were collected after excitation of donor fluorophores at 530 nm and acceptor fluorophores at 630 nm.

*Single-molecule measurements by wide-field fluorescence microscopy*

The 12-well glass slide (Ibidi, Germany) with removable silicon chambers was incubated with 50 $\mu$L of 0.5 mg/mL biotinylated bovine serum albumin solution (Sigma-Aldrich, Buchs, Switzerland) for 15 min at room temperature, washed three times with 1x TAE/12 mM $Mg^{2+}$, and incubated with 50 $\mu$L of 0.5 mg/mL neutravidin solution (Thermo Fisher Scientific, Basel, Switzerland) for 15 min and washed with 1x TAE/12 mM $Mg^{2+}$. Biotinylated DNA origami book samples (100 pM in 1x TAE/12 mM $Mg^{2+}$) were incubated for 15 min and washed three times with 1x TAE/12 mM $Mg^{2+}$. The mix of the oxygen-scavenging system consisted of a 1:1:1 ratio of 1x PCA, 1x PCD, and 1x Trolox mix in 1x TAE/12 mM $Mg^{2+}$ was added into the glass slide chambers where structures were functionalized. In particular, the oxygen-scavenging system was prepared from the stock solutions. The 100x Trolox stock solution contains 100 mg of Trolox, 430 $\mu$L of methanol, and 345 $\mu$L of NaOH (1 M) in 3.2 mL of water. The 40$\times$ PCA stock solution contains 154 mg of PCA in 10 mL of water (pH 9.0). The 100x PCD stock solution contains 9.3 mg of PCD and 13.3 mL of buffer (50% glycerol stock in 50 mM KCl, 1 mM EDTA, and 100 mM Tris-HCl, pH 8.0).

Experiments were performed on a home-built TIRF wide-field microscope, based on an inverted Olympus IX83 body. Laser lines of 532 and 640 nm were used (gem, Laser quantum), filtered by a clean-up filter (ZET532/640x). Dichroic Mirrors (DM) were used to join the path and Pol + $\lambda$/4 is included for circular polarization. The laser light then was focused by two lenses (AC508-100-A-ML And AC254-030-A-ML, Thorlabs, Germany) into the back focal plane of the UPLAPO100x-OHR (1.5 NA, Olympus) objective. After, a Laser Dual Band Set (ET-532/640 nm, DM3) was used to excite the sample and filter the emission simultaneously. The emission was split into two channels using DM4 (T635Ipxr) into an Optosplit II bypass (Cairn) system, to finally reach the chip of the CMOS camera (C14440 ORCA-Fusion, Hamamatsu). To image the structures with FRET-based detection, we used 532 nm laser at 1.0 mW for Cy3, and for the structures with a quenching-based detection, we used lasers 532/640 nm at 1.0 mW for Cy3/Cy5 with direct excitation. After excitation, images were taken every 2 min for 10 min, and data points were collected using two channels the first one for Cy3 emission and the second one for Cy5 emission. For the quenching-based detection mechanism, the direct excitation of Cy3 or Cy5 was collected using the respective channel.

Data analysis was performed on a home-developed Python software. With this software, we were able to find an individual structure, fit them by using a 2D Gaussian model to the center of the section and extract its intensity from all the relevant pixels in each image taken. Energy-transfer efficiency calculations for measurements where the donor is excited and both the donor and acceptor intensities are measured:

$$E = \frac{I_A}{I_A + I_D}$$

where E is energy-transfer efficiency, $I_A$ is acceptor intensity, and $I_D$ is donor intensity.[27]

For quenching-based detection, the increase of fluorescence was calculated in the way that intensity at 0 min period is subtracted from 6 min. The difference between the two intensities was then divided by the 0 min of the same DNA origami book biosensor. The final data point was an increase in intensity over time.

*Isolation of miRNAs*

MCF-7 human breast cancer cells were cultured at 37 °C, 5% $CO_2$, and 95% humidity in Dulbecco's modified Eagle's medium (DMEM) supplemented with Glutamax, 10% fetal bovine serum (FBS), and 1% Penicillin and Streptomycin. All cell culture reagents were purchased from Thermo Fischer Scientific (Basel, Switzerland). MiRNAs were extracted from MCF-7 cells (25 ng/$\mu$L for $10^6$ cells) using a commercial system (High Pure miRNA Isolation Kit; Roche, Switzerland). Extraction was done as described in the kit protocol.

### Example 2

The DNA origami book was designed using caDNAno software (S. M. Douglas, A. H. Marblestone, S. Teerapittayanon, A. Vazquez, G. M. Church and W. M. Shih, Nucleic Acids Res, 2009, 37, 5001-5006. 28Y. Ke, S. M. Douglas, M. Liu, J. Sharma, A. Cheng, A. Leung, Y. Liu, W. M. Shih and H. Yan, J Am Chem Soc, 2009, 131, 15903-15908.). The rectangular structure consists of three layers of DNA helices packed on a square lattice with dimensions of 78.5 x 33.9 x 8 nm (Figure 1a) such that the upper layer consists of two parts connected to the middle layer with a hinge from the centre, allowing the opening of the upper layer at both sides. Each part of the layer has an array of precisely positioned fluorophores (Figure 1, Figure 6).

For FRET-based detection, two arrays of fluorophores were positioned on the upper and middle layers: one array with donor fluorophores on the middle layer facing the upper layer, and the other one on the upper layer with corresponding acceptor fluorophores (Figure 1c). Each array consists of 4 columns of 5 precisely located fluorophores (Figure 1d). For the quenching-based detection approach, the middle layer of the DNA origami book was decorated with an array of different types of fluorophores (Cy3 or Cy5), and the top layer of the book was decorated with quencher molecules (Black Hole Quencher-2 (bh2) and/or Black-Berry Quencher 650 (bbq650)) facing to the corresponding fluorophore in the middle layer (Figure 1d). The distance between each fluorophore on one layer of DNA within a column is 5.8 nm and the distance between two columns is 6.6 nm, according to our design. The mapping of the whole structure can be found in Figure 7.

Partially hybridized locks on both sides constitutively keep the layers in a closed state upon assembly (Figure 1b). Each of the four locks on each side of the book forms 15 bp long duplex where one of the strands is elongated with 8 bases allowing the binding of the complementary target key of 23 bases through toehold-mediated strand displacement. Once bound to the toehold, the target oligonucleotide then displaces the shorter lock strand from the duplex. That promotes the opening of the layers due to the electrostatic repulsion of the DNA and the entropy of the device. The change between open and closed state results in increased distance between donor and acceptor fluorophores causing the decrease in the energy transfer between the fluorophore pairs in both mechanisms (Figure 1c and 1d) and the increase in the fluorescence signal of the donor fluorophores by releasing the respective FRET pair. Transmission electron microscopy (TEM) images demonstrated the formation of both open and closed states of the DNA origami book biosensor (Figure 1e and f, Figure 8). Furthermore, atomic force microscopy (AFM) images performed in scanning mode showed the formation of DNA origami book sensor in a closed state (Figure 9). To further demonstrate the opening of the DNA origami book biosensor, structures were assembled and mixed with one or two DNA oligonucleotide targets and then analysed by agarose gel electrophoresis (Figure 10). The differences in electrophoretic shift (mobility) between structures in closed or open states (one or two sides) were observed, thereby demonstrating the functionality of the DNA origami book biosensor in response to the presence of a corresponding oligonucleotide target.

*Example 3*

*Optimization of the fluorescence output signal*

To check the efficiency of incorporation of the labelled oligonucleotides within the structure, we imaged the DNA origami book structures with 1 column (5 Cy3 or Cy5), 2 columns (10 Cy3 or Cy5), and 4 columns (20 Cy3 or Cy5). The increase in the number of fluorophores incorporated within the structure resulted in a linear increase in the mean fluorescence intensity in the absence of self-quenching (Figure 11). The signal of the DNA origami book biosensor is based on distance-dependent energy transfer between fluorophores, actually between columns of donor and acceptor/quencher. Opening of the structure will result in a bigger distance between the columns of donor and acceptor/quencher pairs that is closer to the locks compared to the distance between the columns closer to the hinge. Our theoretical calculation of possible angles of opening and distances in nanometers between columns is presented in Figure 2a. This calculation indicated that the distance between columns within the structure will be different, and that would result in variations of the FRET. First, we defined the optimal salt concentration for the maximum signal output of the book structure that was subsequently used for the further functional experiments using a spectrophotometer (Figure 12). Then, we studied the FRET efficiency for each column (consisting of 5 donor/acceptor pairs) in the open and close states using a spectrophotometer (Figure 2b). Results confirmed the theoretical calculations that columns closer to the locks have the highest difference in FRET efficiency between open and closed states (30%) while columns close to the hinge have a very low difference in FRET efficiency (1.6%). To determine the reaction time of the DNA origami book biosensor to fully open, the structures were assembled in the close state with a single column of FRET pairs (column 1). To quantify the FRET efficiencies, fluorescence emission profiles of acceptors after excitation of donors of the biosensor device were recorded by spectrophotometer before and every 5 min after the addition of 1 $\mu$M target key over a period of 30 min, and FRET efficiency was calculated (Figure 2c and d). Plotted FRET efficiency showed a decrease over time which indicated that our structure was opening within 10 min after the addition of the target. Longer incubation times did not improve the FRET efficiency significantly. These results were valuable for the subsequent study at the single-structure level.

**Example 4**

*Single-structure analysis of DNA origami book biosensors using wide-field fluorescence microscopy*

Next, we tested the detection specificity of our book biosensor using different targets for the left side and the right side of the structure separately. We initially used DNA analogs of target miRNAs, ODN-153 (for miR-153) and ODN-342 (for miR-342). The structures with 3 columns (1-3) were imaged every 2 min for a total of 10 min with the target added after one minute. Then, the intensity of DNA origami structures was extracted, and FRET efficiency was calculated (Figure 13). As a control experiment, we imaged structures before adding the actual target in the same chamber but at different positions to assess how the laser was affecting the fluorophores and to observe the behaviour of the structures. Our results showed that when non-target DNA is added there is a small change in the mean FRET efficiency for the left (8%) and right (6%) sides of the structure due to exposure to the laser and photobleaching (Figure 3). The addition of the DNA target (ODN-153) for the left side results in a significant change in the average FRET efficiency (26-30%) for concentrations in the range from 1 $\mu$M to 100 pM, while the change for 10 pM was around 10% (Figure 3a). The addition of the DNA target (ODN-342) for the right side in high concentration triggered the opening of the structure faster than the left DNA target. For the concentrations in the range of 1 $\mu$M to 100 pM change in FRET efficiency is approximately 20% while the addition of the 10 pM target resulted in a smaller change of FRET efficiency, 11% (Figure 3b). The reason for the faster opening in higher concentration could be due to different GC% of the target sequence. ODN-342 has higher GC content (52.2%) in comparison to ODN-153 (40.9%) (Table 4). We estimated the theoretical limit of detection (LoD) of both targets based on the measured normalized counts of the non-target oligonucleotide. By fitting all the measured data points ($10^1$ to $10^6$ pM) to an asymmetric five-parameter curve, the LoDs of ODN-153 and ODN-342 were determined at 1.6 pM and 1 pM, respectively. As the DNA origami book biosensor has 4 locks on each side, which keep the structure in a close state, we tested the impact of the number and position of the locks on the detection of the lower concentration of the target of interest using the FRET mechanism of detection. Structures with 3 columns (1-3) were assembled with different numbers (2 or 3) and positions of locks followed by the addition of 100 pM of ODN-153. The FRET changes obtained showed that the incorporation of fewer locks or altering the positioning of the locks within the structure did not increase the mean FRET intensity at the 100 pM concentration of the target of interest (Figure 14).

Furthermore, we tested the detection ability of the book biosensor using the fluorescence quenching mechanism as an alternative FRET-based detection approach. For this, the structures were assembled with 2 columns (1-2) of Cy3 dyes and bh2 quenchers on the left side and imaged every 2 min for a total of 10 min, with the target DNA (ODN-153) added after one minute. The intensity of DNA origami structures was extracted and the change of fluorescence intensity between 0 min and 6 min was calculated. Upon binding of the target of interest, the upper layer opens and the fluorescence intensity of Cy3 increases due to the increased distance between Cy3 and bh2 quencher dyes. The results showed that the addition of ODN-153 in the range of 100 pM to 1 $\mu$M increased the fluorescence intensity (14 to 20%), while the addition of 10 pM increases the fluorescence intensity only by 7%.

The intensity change between closed and open structures can be seen in the histograms (Figure 4a). The same experiment was repeated with book biosensors containing 2 columns (1-2) of Cy5 dyes and bbq650 quenchers on the right side of the structure. Our results showed the addition of ODN-342 in the range of 100 pM to 1 $\mu$M resulted in an increase in the Cy5 fluorescence intensity in the range of 11 to 20%, while the addition of 10 pM target ODN increased the fluorescence intensity by 8% (Figure 4b). By fitting all the measured data points (101 to 106 pM) to an asymmetric five parameter curve, the LoDs of ODN-153 and ODN-342 were determined as 3.3 pM and 3.9 pM, respectively.

**Table 4**

| 296 | TCTCACACAGAAATCGCACCCGT | ODN-342 |
|---|---|---|
| 297 | **TTGCATAGTCACAAAAGTGATC** | ODN-153 |
| 298 | **UGAGGUAGUAGGUUGUAUAGUU** | let-7a |
| 299 | **UCAACAUCAGUCUGAUAAGCUA** | miR-21 |
| 300 | **CATCATAATTTTAAAACAATT** | non-target |

### Example 5

*Multisensing of two target miRNAs and miRNAs extracted from cancer cells*

Next, we performed the multiplex detection of two targets by adding both oligonucleotides simultaneously. The structures were assembled by including 2 columns (1-2) of Cy3/bh2 dye-quencher pairs on the left side and 2 columns (1-2) of Cy5/bbq650 dye-quenchers pairs on the right side. We applied the same procedure described above by adding two target DNAs (ODN-153 and ODN-342) at the same time in the range of 10 pM to 10 nM. Results showed that the fluorescence intensity of both Cy3 and Cy5 dyes were increased (12 to 20%) when targets were added in the concentration range of 100 pM to 10 nM, whereas the fluorescence intensity remained at 10% at the concentration of 10 pM (Figure 15).

Furthermore, to test the RNA detection ability of our system we chose two highly expressed miRNAs (miR-21 and let-7a) by redesigning the locks that are responding to these targets respectively (SEQ ID NO: 280 to 295). As above, the structures were assembled by including 2 columns (1-2) of Cy3/bh2 dye-quencher pairs on the left side and 2 columns (1-2) of Cy5/bbq650 dye-quenchers pairs on the right side. The locks on the left side of the biosensor were designed to detect miR-21 and the locks on the right side were designed to detect let-7a, so that increase in Cy3 fluorescence intensity reveals the detection of miR-21 while an increase in Cy5 fluorescence intensity reveals the detection of let-7a. Testing was done at miRNAs concentrations of 10 nM and 10 pM. Results showed that the fluorescence intensity of Cy3 and Cy5 after the addition of the 10 nM target was increased to 18% to 19% respectively, indicating the concomitant detection of both miRNAs at these concentrations (Figure 5). The fluorescence intensities of both dyes after adding the 10 pM target were approximately 10%.

Finally, in order to validate the capability of our assay to detect cell-derived miRNA in biological samples, we studied with miRNAs from MCF-7 breast cancer cell extracts. MiRNA was extracted from MCF-7 cells (25 ng/$\mu$L for 106 cells) using a commercial system. Then, we added 100 ng of the isolated RNA to our biosensor platform containing single DNA origami structures with locks designed to detect miR-21 and let-7a. Results showed an increase in fluorescence intensity of around 10% for Cy3 and 11% for Cy5, which is in the detection range of our system and above our system's LoD.

In summary, we presented a DNA origami book biosensor for the dual detection of specific target oligonucleotides. Our biosensor offers two different FRET-based mechanisms for the detection: 1) a FRET-based detection system that can reveal the presence of one target of interest and 2) a fluorescence quenching-based detection system that can simultaneously detect two different targets. Within our structure, we can incorporate 20 FRET and/or 20 quenching fluorophores pairs on each side of the book. In both FRET-based detection systems, our book biosensor has a high-intensity output signal at the single molecule level. We demonstrated that our device can detect target oligonucleotides at concentrations as low as 1-10 pM with a detection time of 10 min via both detection mechanisms. Importantly, our approach is highly versatile, as the locks can be easily redesigned to detect different oligonucleotides including different short tumoral ctDNA fragments or miRNAs, viral DNA, or RNA fragments (e.g. SARS-CoV-2, Zika, or HPV), or even longer mRNAs through the combination of multiple locks. When applied in a matrix form it can be expanded to detect hundreds of different targets simultaneously, possibly changing the way oligonucleotide-based diagnostics will be made in the future.

**Claims**

1. A DNA origami biosensor capable of detecting at least two different target oligonucleotides at the same time, said biosensor comprising at least one DNA origami hinge and at least two detection sites, wherein each detection site comprises

   • a signal emitting array,
   • at least one lock comprising a first and a second fastening domain and a toehold domain attached to the first or second fastening domain, wherein the first and the second fastening domain are capable of forming a duplex with each other and wherein the first or second fastening domain is capable of hybridizing to a target oligonucleotide via the toehold domain, wherein the lock is capable of assuming an open or a locked configuration, wherein the lock assumes the open configuration upon hybridization of the target oligonucleotide and the locked configuration in the absence of the target oligonucleotide,
   • at least one movable section, wherein the movable section is connected to the at least one hinge and is movable via said hinge and wherein the position of the movable section depends on the configuration of the lock,

   wherein the movable sections of the detection sites are connected to each other or to a common DNA origami structure via the at least one hinge.

2. The biosensor according to claim 1, wherein in each detection site, the first fastening domain of the lock is attached to the movable section of said detection area and the second fastening domain is attached to the common DNA structure or to the movable section of another detection area.

3. The biosensor according to claim 1 or 2 comprising at least one, preferably two rigid layers as the common DNA origami structure.

4. The biosensor according to claim 3, wherein the biosensor comprises one DNA origami hinge, said hinge being connected to the at least one rigid layer and located in the middle of at least one rigid layer, wherein said hinge is connected to all movable sections.

5. The biosensor according to claim 3, wherein the biosensor comprises two DNA origami hinges connected to the at least one rigid layer, the hinges being located at opposing ends of the at least one rigid layer, wherein each hinge is connected to at least one movable section.

6. The biosensor according to claim 1 or 2, wherein the biosensor comprises three DNA origami hinges and three detection sites, wherein each detection site comprises two movable sections and wherein each hinge is connected to at least two movable sections.

7. The biosensor according to any of claims 1 to 4, wherein the signal emitting array comprises at least one row of donor fluorophores opposing one row of acceptor fluorophores.

8. The biosensor according to any of claims 1 to 4, wherein the signal emitting array comprises at least one row of fluorophores opposing at least one row of quenchers.

9. The biosensor according to any of claims 1 to 8, wherein the target oligonucleotides are selected from the group consisting of miRNA, mRNA, lncRNA, rRNA, DNA and ctDNA.

10. A method for detecting at least two target oligonucleotides in a sample, the method comprising

    a) bringing a biosensor according to any of claims 1 to 9 into contact with the sample.

11. The method according to claim 10, wherein after step a), a secondary probe is added to the sample, the secondary probe being capable of hybridizing to the first or second fastening domain.

12. The method according to claim 10 or 11, wherein during step a), a molecular crowder is added to the sample.

13. A biosensor according to any of claims 1 to 9 for use in diagnosing a disease, wherein the biosensor is capable of detecting target oligonucleotides associated with said disease.

14. The biosensor for use according to claim 11, wherein the disease is associated with altered miRNA expression.

15. The biosensor for use according to claim 14, wherein the disease is selected from the group consisting of cardiovascular diseases, inflammatory diseases autoimmune diseases, neurodegenerative diseases, metabolic conditions, renal diseases, pulmonary diseases, infectious diseases and cancer.

16. The biosensor for use according to claim 15, wherein the disease is cancer and the target oligonucleotide associated with cancer is selected from group of let-7a, miR-139-5p, miR-191, miR-223-3p, miR-376c, let-7b-3p, miR-14, 1-3p miR-192-3p, miR-223-5p, miR-376c-3p, let-7b-5p, miR-141-5p, miR-192-5p, miR-23a, miR-378, let-7c-3p, miR-142-5p, miR-194, miR-23a-3p, miR-382, let-7c-5p, miR-143, miR-195-3p, miR-23b-3p, miR-409-3p, let-7d, miR-145-3p, miR-195-5p, miR-25, miR-411, let-7e, miR-145-5p, miR-196a, miR-26a, miR-421, let-7f miR-146a, miR-198, miR-27a-3p, miR-423-5p, let-7g, miR-146b-3p miR-199a-3p, miR-27a-5p, miR-4257-3p, miR-1, miR-148a, miR-199a-5p, miR-28-3p, miR-451, miR-100, miR-148b, miR-19b-3p, miR-296-5p, miR-4772-3p, miR-100-5p, miR-150, miR-200a, miR-299-5p, miR-486-5p, miR-103, miR-153, miR-200c, miR-29a, miR-497, miR-106a, miR-155-3p, miR-202, miR-29c, miR-601, miR-106b, miR-155-5p, miR-203, miR-301a, miR-625, miR-107, miR-15b, miR-205-3p, miR-30a, miR-7, miR-10a, miR-16, miR-205-5p, miR-30a-3p, miR-718, miR-10b, miR-16-5p, miR-20a-3p, miR-30e-3p, miR-744, miR-10b-3p, miR-17 miR-20a-5p, miR-31, miR-760, miR-122, miR-17-5p, miR-20b-5p, miR-320a, miR-768-3p, miR-1229, miR-18, miR-21, miR-320b, miR-801, miR-1246, miR-181-5p, miR-210-3p, miR-34, miR-885-5p, miR-125a-3p, miR-181a-5p, miR-214, miR-342-5p, miR-92a, miR-126-3p, miR-181b, miR-215, miR-34a, miR-92b, miR-127-3p, miR-181c, miR-216, miR-361-3p, miR-93, miR-1290, miR-182-5p, miR-218, miR-361-5p, miR-9-5p, miR-130, miR-183-5p, miR-22, miR-365, miR-130b, miR-185, miR-221, miR-367, miR-133a, miR-18a, miR-222-3p, miR-372, miR-133b, miR-19, miR-222-5p and miR-375.

**Patentansprüche**

1. Ein DNA Origami-Biosensor, der mindestens zwei verschiedene Zieloligonukleotide gleichzeitig nachweisen kann, wobei der Biosensor mindestens ein DNA Origami-Scharnier und mindestens zwei Nachweisstellen umfasst, wobei jede Nachweisstelle Folgendes umfasst

· eine signalemittierende Anordnung,
· mindestens eine Verriegelung, die eine erste und eine zweite Befestigungsdomäne und eine an die erste oder zweite Befestigungsdomäne gebundene Toehold-Domäne umfasst, wobei die erste und die zweite Befestigungsdomäne in der Lage sind, miteinander ein Duplex zu bilden, und wobei die erste oder zweite Befestigungsdomäne in der Lage ist, über die Toehold-Domäne mit einem Zieloligonukleotid zu hybridisieren, wobei die Verriegelung in der Lage ist, eine offene oder eine verriegelte Konfiguration anzunehmen, wobei die Verriegelung bei Hybridisierung des Zieloligonukleotids die offene Konfiguration und in Abwesenheit des Zieloligonukleotids die verriegelte Konfiguration annimmt,
· mindestens einen beweglichen Abschnitt, wobei der bewegliche Abschnitt mit dem mindestens einen Scharnier verbunden ist und über das Scharnier beweglich ist und wobei die Position des beweglichen Abschnitts von der Konfiguration des Schlosses abhängt,

wobei die beweglichen Abschnitte der Detektionsstellen über das mindestens eine Scharnier miteinander oder mit einer gemeinsamen DNA Origami-Struktur verbunden sind.

2. Der Biosensor nach Anspruch 1, wobei in jeder Detektionsstelle die erste Befestigungsdomäne des Schlosses an dem beweglichen Abschnitt des Detektionsbereichs und die zweite Befestigungsdomäne an der gemeinsamen DNA-Struktur oder an dem beweglichen Abschnitt eines anderen Detektionsbereichs befestigt ist.

3. Der Biosensor nach Anspruch 1 oder 2, der mindestens eine, vorzugsweise zwei starre Schichten als gemeinsame DNA Origami umfasst.

4. Der Biosensor nach Anspruch 3, wobei der Biosensor ein DNA Origami-Scharnier umfasst, wobei das Scharnier mit der mindestens einen starren Schicht verbunden ist und sich in der Mitte der mindestens einen starren Schicht befindet, wobei das Scharnier mit allen beweglichen Abschnitten verbunden ist.

5. Der Biosensor gemäß Anspruch 3, wobei der Biosensor zwei DNA Origami-Scharniere umfasst, die mit der mindestens einen starren Schicht verbunden sind, wobei sich die Scharniere an gegenüberliegenden Enden der mindestens einen starren Schicht befinden, wobei jedes Scharnier mit mindestens einem beweglichen Abschnitt verbunden ist.

6. Der Biosensor nach Anspruch 1 oder 2, wobei der Biosensor drei DNA Origami-Scharniere und drei Detektionsstellen umfasst, wobei jede Detektionsstelle zwei bewegliche Abschnitte umfasst und wobei jedes Scharnier mit mindestens zwei beweglichen Abschnitten verbunden ist.

7. Der Biosensor nach einem der Ansprüche 1 bis 4, wobei die signalemittierende Anordnung mindestens eine Reihe von Donor-Fluorophoren umfasst, die einer Reihe von Akzeptor-Fluorophoren gegenüberliegen.

8. Der Biosensor gemäß einem der Ansprüche 1 bis 4, wobei die signalemittierende Anordnung mindestens eine Reihe von Fluorophoren umfasst, die mindestens einer Reihe von Quenchern gegenüberliegen.

9. Der Biosensor gemäß einem der Ansprüche 1 bis 8, wobei die Zieloligonukleotide aus der Gruppe ausgewählt sind, die aus miRNA, mRNA, lncRNA, rRNA, DNA und ctDNA besteht.

10. Verfahren zum Nachweis von mindestens zwei Zieloligonukleotiden in einer Probe, wobei das Verfahren umfasst

    a) einen Biosensor gemäß einem der Ansprüche 1 bis 9 mit der Probe in Kontakt zu bringen.

11. Verfahren gemäß Anspruch 10, wobei nach Schritt a) eine sekundäre Sonde zu der Probe hinzugefügt wird, wobei die sekundäre Sonde in der Lage ist, an die erste oder zweite Befestigungsdomäne zu hybridisieren.

12. Verfahren nach Anspruch 10 oder 11, wobei während Schritt a) ein molekularer Crowder zu der Probe hinzugefügt wird.

13. Biosensor nach einem der Ansprüche 1 bis 9 zur Verwendung bei der Diagnose einer Krankheit, wobei der Biosensor in der Lage ist, Zieloligonukleotide nachzuweisen, die mit der Krankheit assoziiert sind.

14. Der Biosensor zur Verwendung gemäß Anspruch 11, wobei die Krankheit mit einer veränderten miRNA-Expression assoziiert ist.

15. Der Biosensor zur Verwendung gemäß Anspruch 14, wobei die Krankheit aus der Gruppe ausgewählt ist, die aus Herz-Kreislauf-Erkrankungen, Entzündungskrankheiten, Autoimmunerkrankungen, neurodegenerativen Erkrankungen, Stoffwechselerkrankungen, Nierenerkrankungen, Lungenerkrankungen, Infektionskrankheiten und Krebs besteht.

16. Der Biosensor zur Verwendung nach Anspruch 15, wobei die Krankheit Krebs ist und das mit Krebs assoziierte Zieloligonukleotid ausgewählt ist aus let-7a, miR-139-5p, miR-191, miR-223-3p, miR-376c, let-7b-3p, miR-14, 1-3p miR-192-3p, miR-223-5p, miR-376c-3p, let-7b-5p, miR-141-5p, miR-192-5p, miR-23a, miR-378, let-7c-3p, miR-142-5p, miR-194, miR-23a-3p, miR-382, let-7c-5p, miR-143, miR-195-3p, miR-23b-3p, miR-409-3p, let-7d, miR-145-3p, miR-195-5p, miR-25, miR-411, let-7e, miR-145-5p, miR-196a, miR-26a, miR-421, let-7f miR-146a, miR-198, miR-27a-3p, miR-423-5p, let-7g, miR-146b-3p miR-199a-3p, miR-27a-5p, miR-4257-3p, miR-1, miR-148a, miR-199a-5p, miR-28-3p, miR-451, miR-100, miR-148b, miR-19b-3p, miR-296-5p, miR-4772-3p, miR-100-5p, miR-150, miR-200a, miR-299-5p, miR-486-5p, miR-103, miR-153, miR-200c, miR-29a, miR-497, miR-106a, miR-155-3p, miR-202, miR-29c, miR-601, miR-106b, miR-155-5p, miR-203, miR-301a, miR-625, miR-107, miR-15b, miR-205-3p, miR-30a, miR-7, miR-10a, miR-16, miR-205-5p, miR-30a-3p, miR-718, miR-10b, miR-16-5p, mi-R-20a-3p, miR-30e-3p, miR-744, miR-10b-3p, miR-17, miR-20a-5p, miR-31, miR-760, miR-122, miR-17-5p, miR-20b-5p, miR-320a, miR-768-3p, miR-1229, miR-18, miR-21, miR-320b, miR-801, miR-1246, miR-181-5p, miR-210-3p, miR-34, miR-885-5p, miR-125a-3p, miR-181a-5p, miR-214, miR-342-5p, miR-92a, miR-126-3p, miR-181b, miR-215, miR-34a, miR-92b, miR-127-3p, miR-181c, miR-216, miR-361-3p, miR-93, miR-1290, miR-182-5p, miR-218, miR-361-5p, miR-9-5p, miR-130, miR-183-5p, miR-22, miR-365, miR-130b, miR-185, miR-221, miR-367, miR-133a, miR-18a, miR-222-3p, miR-372, miR-133b, miR-19, miR-222-5p und miR-375.

**Revendications**

1. Biocapteur à origami ADN capable de détecter au moins deux oligonucléotides cibles différents en même temps, ledit biocapteur comprenant au moins une charnière à origami ADN et au moins deux sites de détection, dans lequel chaque site de détection comprend

   · un ensemble émettant un signal,
   · au moins un verrou comprenant un premier et un deuxième domaine de fixation et un domaine d'ancrage fixé au premier ou au deuxième domaine de fixation, dans lequel les premier et deuxième domaines de fixation sont capables de former un duplex l'un avec l'autre et dans lequel le premier ou le deuxième domaine de fixation est capable de s'hybrider à un oligonucléotide cible via le domaine d'ancrage, dans lequel le verrou est capable de prendre une configuration ouverte ou verrouillée, dans lequel le verrou prend la configuration ouverte lors de l'hybridation de l'oligonucléotide cible et la configuration verrouillée en l'absence de l'oligonucléotide cible,
   · au moins une section mobile, dans laquelle la section mobile est reliée à au moins une charnière et est mobile via ladite charnière et dans laquelle la position de la section mobile dépend de la configuration de la serrure,

   dans laquelle les sections mobiles des sites de détection sont reliées les unes aux autres ou à une structure commune en origami ADN via au moins une charnière.

2. Le biocapteur selon la revendication 1, dans lequel, dans chaque site de détection, le premier domaine de fixation de la serrure est attaché à la section mobile de ladite zone de détection et le deuxième domaine de fixation est attaché à la structure d'ADN commune ou à la section mobile d'une autre zone de détection.

3. Le biocapteur selon la revendication 1 ou 2, comprenant au moins une, de préférence deux couches rigides en tant que structure commune en origami ADN.

4. Le biocapteur selon la revendication 3, dans lequel le biocapteur comprend une charnière en origami ADN, ladite charnière étant reliée à la au moins une couche rigide et située au milieu d'au moins une couche rigide, dans lequel ladite charnière est reliée à toutes les sections mobiles.

5. Le biocapteur selon la revendication 3, dans lequel le biocapteur comprend deux charnières en origami ADN reliées à la au moins une couche rigide, les charnières étant situées aux extrémités opposées de la au moins une couche rigide, dans lequel chaque charnière est reliée à au moins une section mobile.

6. Le biocapteur selon la revendication 1 ou 2, dans lequel le biocapteur comprend trois charnières en origami ADN et trois sites de détection, chaque site de détection comprenant deux sections mobiles et chaque charnière étant reliée à au moins deux sections mobiles.

7. Le biocapteur selon l'une quelconque des revendications 1 à **4,** dans lequel l'ensemble émetteur de signaux comprend au moins une rangée de fluorophores donneurs opposée à une rangée de fluorophores accepteurs.

8. Biocapteur selon l'une quelconque des revendications 1 à 4, dans lequel l'ensemble émetteur de signaux comprend au moins une rangée de fluorophores opposée à au moins une rangée de quenchers.

9. Biocapteur selon l'une quelconque des revendications 1 à 8, dans lequel les oligonucléotides cibles sont choisis dans le groupe constitué par les miARN, les ARNm, les ARN lnc, les ARNr, l'ADN et l'ADNct.

10. Procédé de détection d'au moins deux oligonucléotides cibles dans un échantillon, le procédé comprenant

    a) la mise en contact d'un biocapteur selon l'une quelconque des revendications 1 à 9 avec l'échantillon.

11. Procédé selon la revendication 10, dans lequel, après l'étape a), une sonde secondaire est ajoutée à l'échantillon, la sonde secondaire étant capable de s'hybrider au premier ou au deuxième domaine de fixation.

**12.** Procédé selon la revendication 10 ou 11, dans lequel, pendant l'étape a), un agent de saturation moléculaire est ajouté à l'échantillon.

**13.** Biocapteur selon l'une quelconque des revendications 1 à 9 pour utilisation dans le diagnostic d'une pathologie, dans lequel le biocapteur est capable de détecter des oligonucléotides cibles associés à ladite pathologie.

**14.** Biocapteur pour l'utilisation selon la revendication 11, dans lequel la pathologie est associée à une expression altérée des miARN.

**15.** Biocapteur pour l'utilisation selon la revendication 14, dans lequel la pathologie est choisie parmi le groupe comprenant les pathologies cardiovasculaires, les pathologies inflammatoires, les pathologies auto-immunes, les pathologies neurodégénératives, les troubles métaboliques, les pathologies rénales, les pathologies pulmonaires, les pathologies infectieuses et le cancer.

**16.** Le biocapteur pour utilisation selon la revendication 15, dans lequel la pathologie est le cancer et l'oligonucléotide cible associé au cancer est choisi parmi le groupe constitué de let-7a, miR-139-5p, miR-191, miR-223-3p, miR-376c, let-7b-3p, miR-14, 1-3p miR-192-3p, miR-223-5p, miR-376c-3p, let-7b-5p, miR-141-5p, miR-192-5p, miR-23a, miR-378, let-7c-3p, miR-142-5p, miR-194, miR-23a-3p, miR-382, let-7c-5p, miR-143, miR-195-3p, miR-23b-3p, miR-409-3p, let-7d, miR-145-3p, miR-195-5p, miR-25, miR-411, let-7e, miR-145-5p, miR-196a, miR-26a, miR-421, let-7f miR-146a, miR-198, miR-27a-3p, miR-423-5p, let-7g, miR-146b-3p miR-199a-3p, miR-27a-5p, miR-4257-3p, miR-1, miR-148a, miR-199a-5p, miR-28-3p, miR-451, miR-100, miR-148b, miR-19b-3p, miR-296-5p, miR-4772-3p, miR-100-5p, miR-150, miR-200a, miR-299-5p, miR-486-5p, miR-103, miR-153, miR-200c, miR-29a, miR-497, miR-106a, miR-155-3p, miR-202, miR-29c, miR-601, miR-106b, miR-155-5p, miR-203, miR-301a, miR-625, miR-107, miR-15b, miR-205-3p, miR-30a, miR-7, miR-10a, miR-16, miR-205-5p, miR-30a-3p, miR-718, miR-10b, miR-16-5p, miR-20a-3p, miR-30e-3p, miR-744, miR-10b-3p, miR-17, miR-20a-5p, miR-31, miR-760, miR-122, miR-17-5p, miR-20b-5p, miR-320a, miR-768-3p, miR-1229, miR-18, miR-21, miR-320b, miR-801, miR-1246, miR-181-5p, miR-210-3p, miR-34, miR-885-5p, miR-125a-3p, miR-181a-5p, miR-214, miR-342-5p, miR-92a, miR-126-3p, miR-181b, miR-215, miR-34a, miR-92b, miR-127-3p, miR-181c, miR-216, miR-361-3p, miR-93, miR-1290, miR-182-5p, miR-218, miR-361-5p, miR-9-5p, miR-130, miR-183-5p, miR-22, miR-365, miR-130b, miR-185, miR-221, miR-367, miR-133a, miR-18a, miR-222-3p, miR-372, miR-133b, miR-19, miR-222-5p et miR-375.

**a**

78.5 nm    33.9 nm

8 nm

**b**

**c**

**d**

Lock

Quencher

Donor

Lock

1  2  3  4    4  3  2  1

**e**

50 nm

**f**

50 nm

**Figure 1**

Figure 2

Figure 3

Figure 4

Figure 5

Figure 6

Figure 7

**Figure 8**

Figure 9

a

b

Figure 10

Figure 11

**Figure 12**

Figure 13

Figure 14

Figure 15

a DNA_oligo

| # | Time | Type | Area | Height | Width | Area% | Symmetry |
|---|---|---|---|---|---|---|---|
| 1 | 14.023 | MM | 33.3 | 10.3 | 0.0541 | 0.996 | 0.756 |
| 2 | 14.14 | MM | 27.4 | 10.9 | 0.042 | 0.820 | 0.958 |
| 3 | 14.396 | MM | 3283.1 | 521.1 | 0.105 | 98.184 | 1.491 |

b DNA_Cy3

| # | Time | Type | Area | Height | Width | Area% | Symmetry |
|---|---|---|---|---|---|---|---|
| 1 | 15.464 | MM | 25.8 | 5.5 | 0.0776 | 1.207 | 0.85 |
| 2 | 16.372 | MM | 49.2 | 6.6 | 0.124 | 2.307 | 0.686 |
| 3 | 16.663 | MM | 67 | 10.7 | 0.1044 | 3.139 | 1.502 |
| 4 | 17.18 | MM | 1992.8 | 316.9 | 0.1048 | 93.348 | 1.359 |

c DNA_oligo

| # | Time | Type | Area | Height | Width | Area% | Symmetry |
|---|---|---|---|---|---|---|---|
| 1 | 15.476 | BB | 1783.5 | 349.7 | 0.0705 | 100.000 | 0.971 |

d DNA_Cy5

| # | Time | Type | Area | Height | Width | Area% | Symmetry |
|---|---|---|---|---|---|---|---|
| 1 | 15.563 | BV R | 254.3 | 33.4 | 0.1044 | 6.887 | 1.155 |
| 2 | 18.081 | BB | 3438.3 | 467.9 | 0.1177 | 93.113 | 0.885 |

Figure 16

miR-153
miR-342
let-7a
miR-21

Figure 17

miR-153
miR-342
let-7a

Figure 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022072850 A1

**Non-patent literature cited in the description**

- **E. S. ANDERSEN** ; **M. DONG et al.** *Nature*, 2009, vol. 459, 73-76
- **ROTHEMUND, P**. Folding DNA to create nanoscale shapes and patterns. *Nature*, 2006, vol. 440, 297-302
- **DOUGLAS SM** ; **DIETZ H** ; **LIEDL T** ; **HÖGBERG B** ; **GRAF F** ; **SHIH WM**. Self-assembly of DNA into nanoscale three-dimensional shapes.. *Nature*, 2009, vol. 459 (7245), 414-418
- **DOERR, A**. DNA origami in 3D. *Nat Methods*, 2011, vol. 8, 454
- **HAN D** ; **PAL S** ; **NANGREAVE J** ; **DENG Z** ; **LIU Y** ; **YAN H**. DNA Origami with Complex Curvatures in Three-Dimensional Space. *Science*, 2011, vol. 332 (6027), 342-346
- **S. M. DOUGLAS** ; **A. H. MARBLESTONE** ; **S. TEERAPITTAYANON** ; **A. VAZQUEZ** ; **G. M. CHURCH** ; **W. M. SHIH**. *Nucleic Acids Res*, 2009, vol. 37, 5001-5006
- **KE** ; **S. M. DOUGLAS** ; **M. LIU** ; **J. SHARMA** ; **A. CHENG** ; **A. LEUNG** ; **Y. LIU** ; **W. M. SHIH** ; **H. YAN**. *J Am Chem Soc*, 2009, vol. 131, 15903-15908